# EUROPEAN PATENT SPECIFICATION

(11) **EP 4 568 627 B1**
(45) Date of publication and mention of the grant of the patent: **23.07.2025**
(21) Application number: 23808872.8
(22) Date of filing: 09.11.2023
(51) Int. Cl.: A61F 13/0206, A61F 13/0246, A61F 13/05

(54) **A WOUND DRESSING**
WUNDVERBAND
PANSEMENT

(30) Priority: 10.11.2022 US 202263424162 P; 22.12.2022 GB 202219611
(43) Date of publication of application: 18.06.2025
(73) Proprietor: ConvaTec Limited, Deeside, Flintshire CH5 2NU (GB)
(72) Inventor: BALLAMY, Lucy, Deeside Flintshire CH5 2NU (GB); LAY, Amelia, Deeside Flintshire CH5 2NU (GB); GILDING, Duncan, Deeside Flintshire CH5 2NU (GB); JEFFERY, Craig, Deeside Flintshire CH5 2NU (GB)
(74) Representative: Wilson Gunn
(86) International application number: PCT/GB2023/052928
(87) International publication number: WO 2024/100406

(56) References cited:
- EP-B1- 1 962 800
- CN-A- 109 223 312
- GB-A- 800 572

## Description

### Technical Field of the Invention

The present invention relates to a wound dressing. In particular, the invention concerns a negative pressure wound dressing.

### Background to the Invention

Wound dressings are known and are generally suitable for treating a variety of wounds, including chronic and acute wound types, such as infected wounds, venous ulcers, diabetic ulcers, burns and surgical wounds.

Negative pressure has been used to treat a range of chronic and acute wounds. Negative pressure may facilitate wound healing through a number of mechanisms, including removal of excess exudate, reduction in periwound edema and increased perfusion. Combined with the physical forces exerted by th negative pressure which draw the wound edges together, this can result in improved wound outcomes.

Wound dressings typically comprise an adhesive skin contact layer for detachably adhering the dressing to a dermal surface. Conventionally, the adhesive skin contact layer comprises a plurality of perforations (i.e., through holes in the adhesive skin contact layer) which enable fluid, for example wound exudate, to flow through the layer but which prevent tissue ingrowth into other material comprised in the wound dressing. Typically, the perforations have a diameter of no more than 1.2mm to facilitate adhesion of the skin contact layer to the dermal surface and to provide a skin contact layer with sufficient breathability, i.e., allowing water vapour and gas to pass through the layer. Typically, the skin contact layer comprises a silicone adhesive. Disadvantageously, some of the perforations are prone to closing, or at least reducing in diameter, after formation due to the silicone adhesive entering and, therefore, blocking the perforations. While this may increase adhesion of the layer to the dermal surface, this adversely affects the breathability of the skin contact layer, causing an accumulation of bodily fluid at and in proximity to the skin or wound site, therefore, preventing the formation of an environment optimised for wound healing. Conversely, it is known to increase the size of perforations in an adhesive skin contact layer to aid breathability. However, this is achieved at the expense of adhesion of the skin contact layer to a dermal surface and in such cases, the skin contact layer may only weakly adhere to a dermal surface such that movement of a patient while the wound dressing is adhered may cause the wound dressing to partially or completely detach from the patient, adversely affecting the dressing to transmit a negative pressure to the wound site.

Conventional techniques to form the perforations in the adhesive layer include using a hot pin process. However, disadvantageously, this results in the formation of 'slugs' around the perforation (i.e., the material intended to be removed to form the perforation is not wholly disconnected from the main body of material and, therefore, partially obstructs the perforation and results in a non-planar surface of the skin contact layer). To overcome this, ultrasound may be used to create the perforations. However, this process disadvantageously forms doughnut/volcano structure around the perforations.

Portable systems have been developed which include a means to manage the exudate produced by the wound by collecting exudate within the wound dressing, typically in an absorbent material, and by evaporation through the dressing. Such systems mean that a separate collection canister may not be an essential part of the system. An advantage of not needing a canister is that the device is less bulky and more portable. A disadvantage with such devices is that if the dressing exceeds its fluid handling capacity, exudate may be drawn from the absorbent material(s) and enter the pump. The presence of exudate in the pump will eventually cause it to fail and require its replacement. The therapy provided by the system may also be less than optimal due to the potential for excess exudate to collect, or pool, at the wound interface. In order to prevent fouling of the pump with exudate, it is known to provide a barrier layer between the absorbent material and the pump. However, th liquid barrier layer does not improve the ability of the absorbent material(s) to absorb exudate.

Wound contact layers are known to comprise a mixture of gelling and non-gelling fibres. Gelling fibres form a gel upon contact with exudate. However, upon forming a gel, many wound contact layers weaken and in some cases partially dissociate from other layers comprised in an absorbent structure. To overcome this, it is known to provide non-gelling fibres as part of the wound contact surface of the wound contact layer. Such arrangements aim to strike a balance between providing a sufficient amount of gelling fibres and preventing dissociation of the wound contact layer whn it becomes wet with exudate.

However, known wound contact layers fail to adequately strike this balance and such layers either do not adequately prevent pooling of exudate at the wound contact surface or do not adequately adhere to other layers within the wound dressing when the wound contact layer becomes wet with exudate.

During negative pressure therapy, air as well as wound exudate material is removed from a wound site. The wound exudate must be collected remotely from the wound site. Some known negative pressure therapy systems comprise a waste cannister connected to a pump unit for the collection and storage of wound exudate material. However, the use of a canister can result in the therapy system itself being bulky and expensive to manufacture. Also, replacing a canister or a container in a canister in whch wound exudate is collected can be a time consuming and a relatively unhygienic process.

Negative pressure wound dressings have been produced which comprise an absorbent layer to absorb wound exudate in an attempt to remove the need for a cannister and pump system. However, such wound dressings often fail to sufficiently meet two requirements of an absorbent layer in a wound dressing, i.e., that the absorbent layer has a high absorbance capacity and remains flexible (i.e., does not stiffen) when it absorbs wound exudate which subsequently dries.

Known absorbent layers with a relatively low absorbance capacity cause pooling of wound exudate at the wound site. This is both uncomfortable and unhygienic for the patient and for a healthcare professional who replaces the wound dressing. Moreover, there is a risk that the wound and/or surrounding skin will become macerated if in prolonged contact with excess wound exudate.

Moreover, known absorbent layers often become relatively stiff when wound exudate has been absorbed and subsequently dried. This results in the fabric of the absorbent layer 'doming' which, disadvantageously, provides a tunnel effect of air transfer and causes air to leak from the wound dressing, thereby adversely affecting the negative pressure transmitted to the wound site.

Conventional negative pressure wound dressings often comprise relatively rigid dressing and binding components which adversely affects system utility and which provides a relatively rigid wound dressing which is disadvantageous in respect of user comfort. Moreover, such components require individual manufacturing steps to incorporate thm into the wound dressing, therefore, increasing the time of manufacture and cost.

The purpose of such components is to attempt to increase the integrity of the wound dressing to prevent, or at least reduce, dissociation of one or more of the layers comprised therein. However, the advantages of such components often do not outweigh the disadvantages associated with the additional manufacturing steps, complex processing and the rigidity of the final wound dressing. Further, the rigid dressing and binding components often have an adverse effect on the wicking, or movement, of wound exudate through the wound dressing away from the wound site. Additionally, existing negative pressure wound dressing systems do not utilise absorbent materials and additional wound dressing components arranged to maximise the management of wound exudate within the dressing.

It is known to provide a wound dressing comprising a wound contact layer, a transmission layer and an absorbent layer, arranged such that the transmission layer overlies the wound contact layer, and the absorbent layer overlies the transmission layer. This arrangement is disclosed in US 10,507,141. However, such an arrangement does not provide a significant absorbency capacity. This means that the arrangement suffers from unnecessary pooling of wound exudate at the wound site. As such, the hygiene of the wound dressing and of the wound site could be improved which in turn would increase comfort for the user.

CN109223312 discloses a negative pressure wound dressing with a support layer made of warp and weft. The support layer is not in contact with a wound when in use.

GB800572 and EP1962800 each disclose wound dressings having a wound contact layer, but neither are directed to a negative pressure wound dressing.

It is an object of embodiments of th present invention to at least partially overcome or alleviate the above problems and/or to provide an improved wound dressing.

### Summary of the Invention

In a first aspect of the invention, there is provided a negative pressure wound dressing, the dressing comprising a backing layer, an adhesive skin contact layer and an absorbent structure arranged between the backing layer and the adhesive skin contact layer, wherein the adhesive skin contact layer is configured to detachably adhere the dressing to a dermal surface, wherein the backing layer comprises a coupling member configured to connect the dressing to a negative pressure source, wherein the absorbent structure comprises a wound contact layer arranged to contact the wound when the adhesive skin contact layer is adhered to the skin adjacent the wound, the wound contact layer having a first surface and a second surface, wherein the first surface of the wound contact layer contacts the wound in use, wherein the first surface comprises warp stitches, the wound contact layer comprises a plurality of gel-forming fibres, the second surface comprises warp and weft stitches, and the first surface does not comprise any weft stitches.

The warp stitches may comprise any non-gelling fibre.

Thus, the present invention provides a wound contact layer which has a reduced amount of non-gelling materials (for example, non-gelling fibres) at the wound contact surface. Advantageously, this facilitates absorption of wound exudate which, therefore, prevents pooling of exudate at the wound site. As such, an environment optimised for wound healing is maintained at the wound site.

Further advantageously, the present invention provides a wound dressing with greater adhesion between the wound contact layer and other layers of the absorbent structure which may be present, for example a transmission layer positioned between the wound contact layer and an absorbent layer or a backing layer. Thus, the present invention provides a wound dressing which exhibits sufficient strength, without compromising its absorption efficacy, to hold the layers of the wound dressing together even when the dressing is wetted, for example with exudate, and when the dressing is handled.

Thus, the wound contact layer of the present invention beneficially prevents, or at least substantially reduces, pooling of wound exudate at the wound site, maintains an environment optimised for wound healing at the wound site, and exhibits sufficient strength between the layers of the absorbent structure to prevent, or at least substantially reduce the possibility of, separation of the layers of the absorbent structure.

The first surface may comprise between about 0.1 and about 15.0 warp stitches /cm, between about 0.1 and about 12.0 warp stitches /cm, between about 0.1 and about 10.0 warp stitches /cm, between about 0.1 and about 8.0 warp stitches /cm, between about 0.1 and about 6.0 warp stitches /cm, between about 0.1 and about 4.0 warp stitches /cm, between about 0.1 and about 2.0 warp stitches /cm, or between about 0.1 and about 1.0 warp stitches /cm,.

The first surface may comprise between about 0.4 and about 15.0 warp stitches /cm, between about 0.4 and about 12.0 warp stitches /cm, between about 0.4 and about 10.0 warp stitches /cm, between about 0.4 and about 8.0 warp stitches /cm, between about 0.4 and about 6.0 warp stitches /cm, between about 0.4 and about 4.0 warp stitches /cm, between about 0.4 and about 2.0 warp stitches /cm, or between about 0.4 and about 1.0 warp stitches /cm,.

The first surface may comprise between about 1.0 and about 15.0 warp stitches /cm, between about 1.0 and about 12.0 warp stitches /cm, between about 1.0 and about 10.0 warp stitches /cm, between about 1.0 and about 8.0 warp stitches /cm, between about 1.0 and about 6.0 warp stitches /cm, between about 1.0 and about 4.0 warp stitches /cm, or between about 1.0 and about 2.0 warp stitches /cm.

The first surface may comprise between about 2.0 and about 15.0 warp stitches /cm, between about 2.0 and about 12.0 warp stitches /cm, between about 2.0 and about 10.0 warp stitches /cm, between about 2.0 and about 8.0 warp stitches /cm, between about 2.0 and about 6.0 warp stitches /cm, or between about 2.0 and about 4.0 warp stitches /cm, .

The first surface may comprise between about 4.0 and about 15.0 warp stitches /cm, between about 4.0 and about 12.0 warp stitches /cm, between about 4.0 and about 10.0 warp stitches /cm, between about 4.0 and about 8.0 warp stitches /cm, or between about 4.0 and about 6.0 warp stitches /cm.

The first surface may comprise between about 6.0 and about 15.0 warp stitches /cm, between about 6.0 and about 12.0 warp stitches /cm, between about 6.0 and about 10.0 warp stitches /cm, or between about 6.0 and about 8.0 warp stitches /cm.

The first surface may comprise between about 8.0 and about 15.0 warp stitches /cm, between about 8.0 and about 12.0 warp stitches /cm, or between about 8.0 and about 10.0 warp stitches /cm.

The first surface may comprise between about 10.0 and about 15.0 warp stitches /cm, or between about 10.0 and about 12.0 warp stitches /cm.

The first surface may comprise between about 12.0 and about 15.0 warp stitches /cm, or between about 12.0 and about 13.0 warp stitches /cm.

The first surface may comprise between about 14.0 and about 15.0 warp stitches /cm.

The second surface may comprise between about 0.1 and about 15.0 weft stitches /cm, between about 0.1 and about 12.0 weft stitches /cm, between about 0.1 and about 10.0 weft stitches /cm, between about 0.1 and about 8.0 weft stitches /cm, between about 0.1 and about 6.0 weft stitches /cm, between about 0.1 and about 4.0 weft stitches /cm, between about 0.1 and about 2.0 weft stitches /cm, between about 0.1 and about 1.0 weft stitches /cm, or between about 0.1 and about 0.4 weft stitches /cm.

The second surface may comprise between about 1.0 and about 15.0 weft stitches /cm, between about 1.0 and about 12.0 weft stitches /cm, between about 1.0 and about 10.0 weft stitches /cm, between about 1.0 and about 8.0 weft stitches /cm, between about 1.0 and about 6.0 weft stitches /cm, between about 1.0 and about 4.0 weft stitches /cm, between about 1.0 and about 2.0 weft stitches /cm, or between about 1.0 and about 1.5 weft stitches /cm.

The second surface may comprise between about 3.0 and about 15.0 weft stitches /cm, between about 3.0 and about 12.0 weft stitches /cm, between about 3.0 and about 10.0 weft stitches /cm, between about 3.0 and about 8.0 weft stitches /cm, between about 3.0 and about 6.0 weft stitches /cm, between about 3.0 and about 4.0 weft stitches /cm, or between about 3.0 and about 3.5 weft stitches /cm.

The second surface may comprise between about 5.0 and about 15.0 weft stitches /cm, between about 5.0 and about 12.0 weft stitches /cm, between about 5.0 and about 10.0 weft stitches /cm, between about 5.0 and about 8.0 weft stitches /cm, between about 5.0 and about 6.0 weft stitches /cm, or between about 5.0 and about 5.5 weft stitches /cm.

The second surface may comprise between about 6.0 and about 15.0 weft stitches /cm, between about 6.0 and about 12.0 weft stitches /cm, between about 6.0 and about 10.0 weft stitches /cm, between about 6.0 and about 8.0 weft stitches /cm, between about 6.0 and about 7.0 weft stitches /cm, or between about 6.0 and about 6.5 weft stitches /cm.

The second surface may comprise between about 8.0 and about 15.0 weft stitches /cm, between about 8.0 and about 12.0 weft stitches /cm, between about 8.0 and about 10.0 weft stitches /cm, or between about 8.0 and about 9.0 weft stitches /cm.

The second surface may comprise between about 10.0 and about 15.0 weft stitches /cm, between about 10.0 and about 12.0 weft stitches /cm, or between about 10.0 and about 11.0 weft stitches /cm.

The second surface may comprise between about 12.0 and about 15.0 weft stitches /cm, or between about 12.0 and about 13.0 weft stitches /cm.

The second surface may comprise between about 13.0 and about 15.0 weft stitches /cm, between about 13.0 and about 14.5 weft stitches /cm, between about 13.0 and about 14.0 weft stitches /cm, or between about 13.0 and about 13.5 weft stitches /cm.

The second surface may comprise between about 14.0 and about 15.0 weft stitches /cm, or between about 14.0 and about 14.5 weft stitches /cm.

The second surface may comprise between about 0.1 and about 15.0 warp stitches /cm, between about 0.1 and about 12.0 warp stitches /cm, between about 0.1 and about 10.0 warp stitches /cm, between about 0.1 and about 8.0 warp stitches /cm, between about 0.1 and about 6.0 warp stitches /cm, between about 0.1 and about 4.0 warp stitches /cm, between about 0.1 and about 2.0 warp stitches /cm, between about 0.1 and about 1.5 warp stitches /cm, between about 0.1 and about 0.8 warp stitches /cm, or between about 0.1 and about 0.2 warp stitches /cm.

The second surface may comprise between about 1.0 and about 15.0 warp stitches /cm, between about 1.0 and about 12.0 warp stitches /cm, between about 1.0 and about 10.0 warp stitches /cm, between about 1.0 and about 8.0 warp stitches /cm, between about 1.0 and about 6.0 warp stitches /cm, between about 1.0 and about 4.0 warp stitches /cm, between about 1.0 and about 2.0 warp stitches /cm, or between about 1.0 and about 1.5 warp stitches /cm.

The second surface may comprise between about 4.0 and about 15.0 warp stitches /cm, between about 4.0 and about 12.0 warp stitches /cm, between about 4.0 and about 10.0 warp stitches /cm, between about 4.0 and about 8.0 warp stitches /cm, between about 4.0 and about 6.0 warp stitches /cm, between about 4.0 and about 5.0 warp stitches /cm, or between about 4.0 and about 4.5 warp stitches /cm.

The second surface may comprise between about 6.0 and about 15.0 warp stitches /cm, between about 6.0 and about 12.0 warp stitches /cm, between about 6.0 and about 10.0 warp stitches /cm, between about 6.0 and about 8.0 warp stitches /cm, between about 6.0 and about 7.0 warp stitches /cm, or between about 6.0 and about 6.5 warp stitches /cm.

The second surface may comprise between about 8.0 and about 15.0 warp stitches /cm, between about 8.0 and about 12.0 warp stitches /cm, between about 8.0 and about 10.0 warp stitches /cm, between about 8.0 and about 9.0 warp stitches /cm, or between about 8.0 and about 8.5 warp stitches /cm.

The second surface may comprise between about 10.0 and about 15.0 warp stitches /cm, between about 10.0 and about 12.0 warp stitches /cm, between about 10.0 and about 11.0 warp stitches /cm, or between about 10.0 and about 10.5 warp stitches /cm.

The second surface may comprise between about 12.0 and about 15.0 warp stitches /cm, between about 12.0 and about 13.0 warp stitches /cm, or between about 12.0 and about 12.5 warp stitches /cm.

The second surface may comprise between about 13.0 and about 15.0 warp stitches /cm, between about 13.0 and about 14.0 warp stitches /cm, or between about 13.0 and about 13.5 warp stitches /cm.

The second surface may comprise between about 14.0 and about 15.0 warp stitches /cm, or between about 14.0 and about 14.5 warp stitches /cm.

Advantageously, in the wound dressing of the invention, the amount of non-gelling fibres (which may include weft stitches) at the wound contact surface is minimised. Thus, there is a greater ratio of gelling fibres to non-gelling fibres at the wound contact surface in contact with the wound and, therefore, wound exudate. Thus, the absorption capacity of the wound contact layer is increased. As such, pooling of wound exudate at the wound site is prevented, or at least significantly reduced.

The second surface may comprise fewer than 15.0 weft stitches /cm, fewer than 13.0 weft stitches /cm, fewer than 11.0 weft stitches /cm, fewer than 9.0 weft stitches /cm, fewer than 7.0 weft stitches /cm, fewer than 5.0 weft stitches /cm, fewer than 3.0 weft stitches /cm, fewer than 1.0 weft stitch /cm, fewer than 0.8 weft stitches /cm, fewer than 0.6 weft stitches /cm, fewer than 0.5 weft stitches /cm, fewer than 0.4 weft stitches /cm, fewer than 0.3 weft stitches /cm, fewer than 0.2 weft stitches /cm, or fewer than 0.1 weft stitch /cm.

The second surface may comprise about 15.0 weft stitches /cm, about 14.5 weft stitches /cm, about 14.0 weft stitches /cm, about 13.5 weft stitches /cm, about 13.0 weft stitches /cm, about 12.5 weft stitches /cm, about 12.0 weft stitches /cm, about 11.5 weft stitches /cm, about 11.0 weft stitches /cm, about 10.5 weft stitches /cm, about 10.0 weft stitches /cm, about 9.5 weft stitches /cm, about 9.0 weft stitches /cm, about 8.5 weft stitches /cm, about 8.0 weft stitches /cm, about 7.5 weft stitches /cm, about 7.0 weft stitches /cm, about 6.5 weft stitches /cm, about 6.0 weft stitches /cm, about 5.5 weft stitches /cm, about 5.0 weft stitches /cm, about 4.5 weft stitches /cm, about 4.0 weft stitches /cm, about 3.5 weft stitches /cm, about 3.0 weft stitches /cm, about 2.5 weft stitches /cm, about 2.0 weft stitches /cm, about 1.5 weft stitches /cm, about 1.0 weft stitch /cm, about 0.8 weft stitches /cm, about 0.6 weft stitches /cm, about 0.5 weft stitches /cm, about 0.4 weft stitches /cm, about 0.3 weft stitches /cm, about 0.2 weft stitches /cm, or about 0.1 weft stitch /cm.

Weft stitches generally impart strength to the wound contact layer fabric in the transverse direction. As such, advantageously, a second surface comprising weft stitches increases the strength of the wound contact layer fabric in a transverse direction. This prevents, or at least significantly reduces the possibility of, dissociation of the wound contact layer when it becomes wet with exudate.

The wound contact layer may have a peripheral region and a central region. The peripheral region may wholly, or at least partially, surround the central region. In embodiments comprising an adhesive skin contact layer comprising a window, the central region of the wound contact layer may be arranged within the window of the adhesive skin contact layer.

The first surface may have a peripheral region and a central region. The peripheral region may wholly, or at least partially, surround the central region.

The second surface may have a peripheral region and a central region. The peripheral region may wholly, or at least partially, surround the central region.

The peripheral region of the second surface may comprise warp stitches only and the central region of the second surface may comprise warp and weft stitches.

The central region of the second surface may comprise warp stitches only and the peripheral region of the second surface may comprise warp and weft stitches.

The peripheral region and the central region of the second surface may comprise warp and weft stitches.

The peripheral region of the first surface and of the second surface may comprise warp stitches only.

The wound contact layer may have a weight of between about 40gsm and about 250gsm, about 40gsm and about 240gsm, about 45gsm and about 230gsm, about 50gsm and about 220gsm, about 55gsm and about 210gsm, about 60gsm and about 200gsm, about 65gsm and about 190gsm, about 70gsm and about 180gsm, about 75gsm and about 170gsm, about 80gsm and about 160gsm, about 85gsm and about 150gsm, about 90gsm and about 140gsm, about 92gsm and about 130gsm, about 94gsm and about 120gsm, about 96gsm and about 110gsm, about 98gsm and about 105gsm, or about 100gsm.

Advantageously, a wound contact layer having a weight of between about 40gsm and about 250gsm, for example about 100gsm, means that the wound contact layer has sufficient strength such that it does not deteriorate in the wound dressing during use and it is not too thick to process during manufacture of the wound dressing, for example when passing the layer through a stitch-bonding machine.

The wound contact layer may have a weight of about 40gsm, about 45gsm, about 50gsm, about 55gsm, about 60gsm, about 65gsm, about 70gsm, about 75gsm, about 80gsm, about 85gsm, about 90gsm, about 95gsm, about 100gsm, about 105gsm, about 110gsm, about 115gsm, about 120gsm, about 125gsm, about 130gsm, about 135gsm, about 140gsm, about 145gsm, about 150gsm, about 155gsm, about 160gsm, about 165gsm, about 170gsm, about 175gsm, about 180gsm, about 185gsm, about 190gsm, about 195gsm, about 200gsm, about 205gsm, about 210gsm, about 215gsm, about 220gsm, about 225gsm, about 230gsm, about 235gsm, about 240gsm, about 245gsm, or about 250gsm.

The wound contact layer may have a weight of at least about 40gsm, about 45gsm, about 50gsm, about 55gsm, about 60gsm, about 65gsm, about 70gsm, about 75gsm, about 80gsm, about 85gsm, about 90gsm, about 95gsm, about 100gsm, about 105gsm, about 110gsm, about 115gsm, about 120gsm, about 125gsm, about 130gsm, about 135gsm, about 140gsm, about 145gsm, about 150gsm, about 155gsm, about 160gsm, about 165gsm, about 170gsm, about 175gsm, about 180gsm, about 185gsm, about 190gsm, about 195gsm, about 200gsm, about 205gsm, about 210gsm, about 215gsm, about 220gsm, about 225gsm, about 230gsm, about 235gsm, about 240gsm, about 245gsm, or at least about 250gsm.

The wound contact layer may have a weight of no more than about 40gsm, about 45gsm, about 50gsm, about 55gsm, about 60gsm, about 65gsm, about 70gsm, about 75gsm, about 80gsm, about 85gsm, about 90gsm, about 95gsm, about 100gsm, about 105gsm, about 110gsm, about 115gsm, about 120gsm, about 125gsm, about 130gsm, about 135gsm, about 140gsm, about 145gsm, about 150gsm, about 155gsm, about 160gsm, about 165gsm, about 170gsm, about 175gsm, about 180gsm, about 185gsm, about 190gsm, about 195gsm, about 200gsm, about 205gsm, about 210gsm, about 215gsm, about 220gsm, about 225gsm, about 230gsm, about 235gsm, about 240gsm, about 245gsm, or no more than about 250gsm.

The wound contact layer may have a weight of between about 40 gsm and about 250 gsm, between about 40 gsm and about 210 gsm, between about 40 gsm and about 170 gsm, between about 40 gsm and about 130 gsm, between about 40 gsm and about 90 gsm, between about 40 gsm and about 70 gsm, or between about 40 gsm and about 50 gsm.

The wound contact layer may have a weight of between about 60 gsm and about 250 gsm, between about 60 gsm and about 210 gsm, between about 60 gsm and about 170 gsm, between about 60 gsm and about 130 gsm, between about 60 gsm and about 90 gsm, or between about 60 gsm and about 70 gsm.

The wound contact layer may have a weight of between about 80 gsm and about 250 gsm, between about 80 gsm and about 210 gsm, between about 80 gsm and about 170 gsm, between about 80 gsm and about 130 gsm, between about 80 gsm and about 110 gsm, or between about 80 gsm and about 90 gsm.

The wound contact layer may have a weight of between about 100 gsm and about 250 gsm, between about 100 gsm and about 210 gsm, between about 100 gsm and about 170 gsm, between about 100 gsm and about 130 gsm, or between about 100 gsm and about 110 gsm.

The wound contact layer may have a weight of between about 120 gsm and about 250 gsm, between about 120 gsm and about 210 gsm, between about 120 gsm and about 170 gsm, or between about 120 gsm and about 130 gsm.

The wound contact layer may have a weight of between about 140 gsm and about 250 gsm, between about 140 gsm and about 210 gsm, between about 140 gsm and about 170 gsm, or between about 140 gsm and about 150 gsm.

The wound contact layer may have a weight of between about 160 gsm and about 250 gsm, between about 160 gsm and about 210 gsm, or between about 160 gsm and about 170 gsm.

The wound contact layer may have a weight of between about 180 gsm and about 250 gsm, between about 180 gsm and about 210 gsm, or between about 180 gsm and about 190 gsm.

The wound contact layer may have a weight of between about 200 gsm and about 250 gsm, between about 200 gsm and about 230 gsm, or between about 200 gsm and about 210 gsm.

The wound contact layer may have a weight of between about 220 gsm and about 250 gsm, or between about 220 gsm and about 230 gsm.

The wound contact layer may have a weight of between about 240 gsm and about 250 gsm.

The wound contact layer may have a thickness of between about 1 mm and about 5 mm, about 1.5 mm and about 4.5 mm, about 2.0 mm and about 4.0 mm, about 2.5 mm and about 3.5 mm, or about 3.0 mm.

The wound contact layer may have a thickness of about 1 mm, about 1.5 mm, about 2.0 mm, about 2.5 mm, about 3.0 mm, about 3.5 mm, about 4.0 mm, about 4.5 mm, or about 5.0 mm.

The warp stitches comprised in the first surface may have a pitch of between about 1.0mm and about 30.0mm, about 2.0mm and about 20.0mm, about 4.0mm and about 8.0mm, about 5.0mm and about 7.0mm, about 6.0mm and about 7.0mm, about 6.1mm and about 6.9mm, about 6.2mm and about 6.8mm, about 6.3mm and about 6.7mm, about 6.4mm and about 6.7mm, about 6.5mm and about 6.7mm, or about 6.6mm.

The warp stitches comprised in the first surface may have a pitch of between about 4.0mm and about 30.0mm, between about 4.0mm and about 25.0mm, between about 4.0mm and about 20.0mm, between about 4.0mm and about 15.0mm, between about 4.0mm and about 10.0mm, between about 4.0mm and about 8.0mm, or between about 4.0mm and about 6.0mm.

The warp stitches comprised in the first surface may have a pitch of between about 8.0mm and about 30.0mm, between about 8.0mm and about 25.0mm, between about 8.0mm and about 20.0mm, between about 8.0mm and about 15.0mm, or between about 8.0mm and about 10.0mm.

The warp stitches comprised in the first surface may have a pitch of between about 15.0mm and about 30.0mm, between about 15.0mm and about 25.0mm, or between about 15.0mm and about 20.0mm.

The warp stitches comprised in the first surface may have a pitch of between about 20.0mm and about 30.0mm, or between about 20.0mm and about 25.0mm.

The warp stitches comprised in the first surface may have a pitch of between about 25.0mm and about 30.0mm.

The warp stitches comprised in the second surface may have a pitch of between about 1.0mm and about 30.0mm, about 1.2mm and about 28.0mm, about 2.0mm and about 20.0mm, about 2.4mm and about 16.0mm, about 2.8mm and about 12.0mm, about 3.0mm and about 10.0mm, about 3.6mm and about 8.8mm, about 4.2mm and about 7.8mm, about 4.8mm and about 7.2mm, about 5.4mm and about 7.0mm, about 5.6mm and about 7.0mm, about 5.8mm and about 7.0mm, about 6.0mm and about 7.0mm, about 6.1mm and about 6.9mm, about 6.2mm and about 6.8mm, about 6.3mm and about 6.7mm, about 6.4mm and about 6.7mm, about 6.5mm and about 6.7mm, or about 6.6mm.

The warp stitches comprised in the second surface may have a pitch of between about 4.0mm and about 30.0mm, between about 4.0mm and about 20.0mm, between about 4.0mm and about 10.0mm, between about 4.0mm and about 8.0mm, or between about 4.0mm and about 6.0mm.

The warp stitches comprised in the second surface may have a pitch of between about 6.0mm and about 30.0mm, between about 6.0mm and about 20.0mm, between about 6.0mm and about 10.0mm, or between about 6.0mm and about 8.0mm.

Advantageously, the pitch of the warp stitches is of sufficient distance to allow gelling fibres to be the main contact surface of the wound contact layer upon the wound, and is sufficiently narrow enough to maintain wet tensile strength during use, therefore, preventing deterioration of the wound contact layer when it becomes wetted during use.

In embodiments comprising a surface having warp and weft stitches, the weft stitches are laid in between the warp stitches and 'knotted' to the warp stitches at anchor points. The anchor points may be at intervals of about 1mm, about 2mm, about 3mm, about 4mm, about 5mm, about 6mm, about 7mm, about 8mm, about 9mm, or about 10mm. The anchor points may be at intervals of between about 1mm and about 10mm, about 2mm and about 9mm, about 3mm and about 8mm, about 4mm and about 7mm, about 5mm and about 6.5mm, or about 6mm.

The wound contact layer may comprise a plurality of gel-forming fibres. By gel-forming fibres is meant hygroscopic fibres which upon the uptake of wound exudate become moist slippery or gelatinous. The gel-forming fibres may be of the type which retain their structural and fibrous integrity on absorption of exudate and, therefore retain a significant amount of tensile strength, or may be of the type which lose their fibrous form and become an amorphous or structureless gel. The gel-forming fibres may be sodium carboxymethylcellulose fibres, chemically modified cellulosic fibres, alkyl sulphonate modified cellulosic fibres, pectin fibres, alginate fibres, chitosan fibres, hyaluronic acid fibres, or other polysaccharide fibres or fibres derived from gums or plants, or combinations or blends of these fibres.

The wound contact layer may comprise a combination or blend of gelling fibres and non-gelling fibres. The gelling fibres and the non-gelling fibres may be any such fibres disclosed herein. For example, the wound contact layer may comprise a combination or blend of fibres comprising a gelling fibre such as fibres formed of sodium carboxymethyl cellulose. The wound contact layer may comprise a combination or blend of fibres comprising a non-gelling fibre such as cellulose fibres.

The gelling fibres may be present in the combination or blend of fibres in an amount equal to about 10%, about 20%, about 30%, about 40%, about 50%, about 60%, about 70%, about 80%, or about 90% of the total mass of the combination or blend of fibres.

The gelling fibres may be present in the combination or blend of fibres in an amount of at least about 10%, about 20%, about 30%, about 40%, about 50%, about 60%, about 70%, about 80%, or at least about 90% of the total mass of the combination or blend of fibres.

The gelling fibres may be present in the combination or blend of fibres in an amount of no more than about 10%, about 20%, about 30%, about 40%, about 50%, about 60%, about 70%, about 80%, or no more than about 90% of the total mass of the combination or blend of fibres.

The gelling fibres may be present in the combination or blend of fibres in an amount of between about 5% and about 95%, about 10% and about 90%, about 15% and about 85%, about 20% and about 80%, about 25% and about 75%, about 30% and about 70%, about 35% and about 65%, about 40% and about 60%, about 45% and about 55%, or about 50% of the total mass of the combination or blend of fibres.

The non-gelling fibres may be present in the combination or blend of fibres in an amount equal to about 10%, about 20%, about 30%, about 40%, about 50%, about 60%, about 70%, about 80%, or about 90% of the total mass of the combination or blend of fibres.

The non-gelling fibres may be present in the combination or blend of fibres in an amount of at least about 10%, about 20%, about 30%, about 40%, about 50%, about 60%, about 70%, about 80%, or at least about 90% of the total mass of the combination or blend of fibres.

The non-gelling fibres may be present in the combination or blend of fibres in an amount of no more than about 10%, about 20%, about 30%, about 40%, about 50%, about 60%, about 70%, about 80%, or no more than about 90% of the total mass of the combination or blend of fibres.

The non-gelling fibres may be present in the combination or blend of fibres in an amount of between about 5% and about 95%, about 10% and about 90%, about 15% and about 85%, about 20% and about 80%, about 25% and about 75%, about 30% and about 70%, about 35% and about 65%, about 40% and about 60%, about 45% and about 55%, or about 50% of the total mass of the combination or blend of fibres.

The wound contact layer may comprise a combination or blend of fibres comprising fibres formed of sodium carboxymethyl cellulose in an amount of greater than or equal to about 50% of the total mass of the combination or blend of fibres, and cellulose fibres in an amount of less than or equal to about 50% of the total mass of the combination or blend of fibres. The sodium carboxymethyl cellulose fibres may be present in an amount equal to about 55% and the cellulose fibres may be present in an amount equal to about 45%, the sodium carboxymethyl cellulose fibres may be present in an amount equal to about 60% and the cellulose fibres may be present in an amount equal to about 40%, the sodium carboxymethyl cellulose fibres may be present in an amount equal to about 65% and the cellulose fibres may be present in an amount equal to about 35%, the sodium carboxymethyl cellulose fibres may be present in an amount equal to about 70% and the cellulose fibres may be present in an amount equal to about 30%, the sodium carboxymethyl cellulose fibres may be present in an amount equal to about 75% and the cellulose fibres may be present in an amount equal to about 25%, the sodium carboxymethyl cellulose fibres may be present in an amount equal to about 80% and the cellulose fibres may be present in an amount equal to about 20%, the sodium carboxymethyl cellulose fibres may be present in an amount equal to about 85% and the cellulose fibres may be present in an amount equal to about 15%, the sodium carboxymethyl cellulose fibres may be present in an amount equal to about 90% and the cellulose fibres may be present in an amount equal to about 10%, or the sodium carboxymethyl cellulose fibres may be present in an amount equal to about 95% and the cellulose fibres may be present in an amount equal to about 5%, of the total mass of the combination or blend of fibres.

The combination or blend of gelling and non-gelling fibres may be blended into a non-woven fabric. The non-woven fabric may be stitchbonded as described herein. In embodiments comprising sodium carboxymethylcellulose fibres, the fibres may have a degree of substitution of between about 0.05 carboxymethyl groups per glucose unit and about 0.50 carboxymethyl groups per glucose unit, about 0.10 carboxymethyl groups per glucose unit and about 0.45 carboxymethyl groups per glucose unit, about 0.15 carboxymethyl groups per glucose unit and about 0.40 carboxymethyl groups per glucose unit, about 0.20 carboxymethyl groups per glucose unit and about 0.35 carboxymethyl groups per glucose unit, about 0.25 carboxymethyl groups per glucose unit and about 0.35 carboxymethyl groups per glucose unit, or about 0.30 carboxymethyl groups per glucose unit.

Advantageously, this means that the sodium carboxymethylcellulose fibres are relatively high-gelling, i.e., more absorbent and retentive, than fibres often comprised in wound contact layers of the prior art. As such, the wound contact layer is capable of absorbing and retaining a greater amount of wound exudate, without adversely affecting its adhesion to other layers within the absorbent structure, compared to wound contact layers which do not comprise sodium carboxymethylcellulose fibres. Thus, pooling of wound exudate at the wound site is prevented, or at least significantly reduced.

Advantageously, due to the absence of weft stitches in the first surface, the gel-forming fibres are in contact with the wound to a greater extent than in wound dressings of the prior art which comprise a wound contact layer having warp and weft stitches on the wound contact surface. As such, the wound contact layer of the present invention absorbs a substantially greater amount of wound exudate compared to wound contact layers of the prior art.

In embodiments comprising sodium carboxymethylcellulose fibres, the fibres may have a degree of substitution of about 0.05 carboxymethyl groups per glucose unit, about 0.10, about 0.15, about 0.20, about 0.25, about 0.30, about 0.35, about 0.40, about 0.45, or about 0.50 carboxymethyl groups per glucose unit.

In embodiments comprising sodium carboxymethylcellulose fibres, the fibres may have a degree of substitution of at least about 0.05 carboxymethyl groups per glucose unit, about 0.10, about 0.15, about 0.20, about 0.25, about 0.30, about 0.35, about 0.40, about 0.45, or at least about 0.50 carboxymethyl groups per glucose unit.

In embodiments comprising sodium carboxymethylcellulose fibres, the fibres may have a degree of substitution of no more than about 0.05 carboxymethyl groups per glucose unit, about 0.10, about 0.15, about 0.20, about 0.25, about 0.30, about 0.35, about 0.40, about 0.45, or no more than about 0.50 carboxymethyl groups per glucose unit.

In embodiments comprising sodium carboxymethylcellulose fibres, the fibres may have a degree of substitution of between about 0.10 carboxymethyl groups per glucose unit and about 0.50 carboxymethyl groups per glucose unit, between about 0.10 carboxymethyl groups per glucose unit and about 0.45 carboxymethyl groups per glucose unit, between about 0.10 carboxymethyl groups per glucose unit and about 0.40 carboxymethyl groups per glucose unit, between about 0.10 carboxymethyl groups per glucose unit and about 0.35 carboxymethyl groups per glucose unit, between about 0.10 carboxymethyl groups per glucose unit and about 0.30 carboxymethyl groups per glucose unit, between about 0.10 carboxymethyl groups per glucose unit and about 0.25 carboxymethyl groups per glucose unit, between about 0.10 carboxymethyl groups per glucose unit and about 0.20 carboxymethyl groups per glucose unit, or between about 0.10 carboxymethyl groups per glucose unit and about 0.15 carboxymethyl groups per glucose unit.

In embodiments comprising sodium carboxymethylcellulose fibres, the fibres may have a degree of substitution of between about 0.20 carboxymethyl groups per glucose unit and about 0.50 carboxymethyl groups per glucose unit, between about 0.20 carboxymethyl groups per glucose unit and about 0.45 carboxymethyl groups per glucose unit, between about 0.20 carboxymethyl groups per glucose unit and about 0.40 carboxymethyl groups per glucose unit, between about 0.20 carboxymethyl groups per glucose unit and about 0.35 carboxymethyl groups per glucose unit, between about 0.20 carboxymethyl groups per glucose unit and about 0.30 carboxymethyl groups per glucose unit, or between about 0.20 carboxymethyl groups per glucose unit and about 0.25 carboxymethyl groups per glucose unit.

In embodiments comprising sodium carboxymethylcellulose fibres, the fibres may have a degree of substitution of between about 0.30 carboxymethyl groups per glucose unit and about 0.50 carboxymethyl groups per glucose unit, between about 0.30 carboxymethyl groups per glucose unit and about 0.45 carboxymethyl groups per glucose unit, between about 0.30 carboxymethyl groups per glucose unit and about 0.40 carboxymethyl groups per glucose unit, or between about 0.30 carboxymethyl groups per glucose unit and about 0.35 carboxymethyl groups per glucose unit.

In embodiments comprising sodium carboxymethylcellulose fibres, the fibres may have a degree of substitution of between about 0.40 carboxymethyl groups per glucose unit and about 0.50 carboxymethyl groups per glucose unit, or between about 0.40 carboxymethyl groups per glucose unit and about 0.45 carboxymethyl groups per glucose unit.

The degree of substitution of the sodium carboxymethylcellulose fibres can be measured by IR spectroscopy (as defined in WO00/01425).

In embodiments comprising sodium carboxymethylcellulose fibres, the fibres may be made by carboxymethylating a woven or non-woven cellulosic fabric such that the absorbency is increased. The woven or non-woven cellulosic fabric may have an absorbency of between about 10g/g of physiological saline solution (Solution A, a sodium/calcium chloride solution described below) to about 30g/g of Solution A, about 15g/g of Solution A to about 25g/g of Solution A, about 15g/g of Solution A to about 20g/g of Solution A, or about 18g/g of Solution A, as measured by the method described in BS EN 13726-1 (2002) "Test methods for primary wound dressings", section 3.2 "Free swell absorptive capacity". The carboxymethylation is generally performed by contacting the fabric with an alkali and a carboxymethylating agent such as chloroacetic acid in an aqueous system.

Solution A, as described in BS EN 13726-1 (2002), consists of sodium chloride and calcium chloride solution containing 142 mmol of sodium ions and 2.5 mmol of calcium ions as the chloride salts. This solution has an ionic composition comparable to human serum or wound exudate. It is prepared by dissolving 8.298 g of sodium chloride and 0.368 g of calcium chloride dihydrate in deionised water and making up to 1 litre in a volumetric flask.

The wound contact layer may comprise a proportion of non-cellulosic textile fibres or gel forming fibres, or non gel-forming fibres. The fabric may comprise fibres textiled into a non-woven fabric.

The gel-forming fibres may have an absorbency of at least 1 gram 0.9% saline solution per gram of fibre (as measured by the free swell method).

The wound contact layer may be stitched using lyocell yarn, cotton, nylon, polyester, or any non-gelling fibre. In particular, the wound contact layer may be stitched using lyocell yarn because, advantageously, this results in highly aligned cellulose fibres having a high degree of crystallinity, making the cellulose fibres collectively stronger so they are able to withstand manufacturing processes and provide a stronger wound contact layer which does not deteriorate in use. This also enhances the strength of the absorbent structure of the wound dressing.

The wound contact layer may, for example, be a Hydrofiber (RTM) material available from ConvaTec Ltd of Deeside.

The source of negative pressure may be capable of generating a minimum of 40mmHg and a maximum of 200mmHg, for example 125mmHg, at the wound site.

The wound contact layer may comprise multiple layers bonded together, for example the wound contact layer may comprise two or more, for example three, four, five or six, layers of fabric bonded together. Each layer may have a weight of about 100gsm, or any weight defined above. The multiple layers may be bonded together by stitch bonding or lamination. For example, the wound contact layer may comprise two layers, each having a weight of 100gsm, stitch bonded together, such that the wound contact layer has a total weight of 200gsm.

In embodiments comprising a wound contact layer comprising multiple layers, for example a first layer and a second layer, each layer may comprise a first surface and a second surface. The first surface of the first layer is arranged to contact the wound in use. The second surface of the first layer is arranged facing the first surface of the second layer. The second surface of the first layer, and the first and second surfaces of the second layer, may comprise warp and weft stitches. The first and second surfaces of the first and second layers may comprise any features, including any optional features, mentioned above.

The superabsorbent material may comprise sodium polyacrylate. The sodium polyacrylate may be a powder, foam, sponge or fibres.

According to an embodiment, there is provided a method for manufacturing a negative pressure wound dressing comprising the steps:
a. Providing an adhesive skin contact layer and a backing layer;
b. Providing a wound contact layer, a transmission layer and a superabsorbent layer, which together form an absorbent structure;
c. Adhering together at least two of the wound contact layer, the transmission layer and the superabsorbent layer; and
d. Arranging the absorbent structure between the adhesive skin contact layer and the backing layer.

The transmission layer may be arranged between the wound contact layer and the superabsorbent layer.

In step c, the at least two of the wound contact layer, the transmission layer and the superabsorbent layer may be adhered together by lamination.

In step c, each of the wound contact layer, the transmission layer and the superabsorbent layer may be adhered together by lamination.

Lamination may be by a hot melt, scatter-coat adhesive.

The at least two of the wound contact layer, the transmission layer and the superabsorbent layer may be laminated by heat lamination.

The at least two of the wound contact layer, the transmission layer and the superabsorbent layer may be laminated by heat lamination using a heat laminator.

The at least two of the wound contact layer, the transmission layer and the superabsorbent layer may be laminated by a laminator having a heating zone temperature between about 50°C and about 200°C, about 70°C and about 180°C, about 80°C and about 170°C, about 100°C and about 150°C, about 120°C and about 130°C, or about 125°C.

The at least two of the wound contact layer, the transmission layer and the superabsorbent layer may be laminated by a laminator having a cooling zone temperature of between about 0°C and about 50°C, about 10°C and about 40°C, about 20°C and about 30°C, or about 25°C.

The at least two of the wound contact layer, the transmission layer and the superabsorbent layer may be laminated by a laminator having a web tension (for each unwind reel) of between about 10 N and about 240 N, about 30 N and about 220 N, about 50 N and about 200 N, about 70 N and about 180 N, about 90 N and about 160 N, about 110 N and about 140 N, about 120 N and about 130 N, or about 125 N.

The at least two of the wound contact layer, the transmission layer and the superabsorbent layer may be laminated by a laminator having a crush roller pressure of about 10 N, about 50 N, about 100 N, about 150 N, about 200 N, about 250 N, about 300 N, about 350 N, about 400 N, about 450 N, about 500 N, about 550 N, about 600 N, about 650 N, about 700 N, about 750 N, about 800 N, about 850 N, about 900 N, about 950 N, or about 1000 N.

The at least two of the wound contact layer, the transmission layer and the superabsorbent layer may be laminated by a laminator having a web speed of about 1 m/min, about 2 m/min, about 3 m/min, about 4 m/min, about 5 m/min, about 6 m/min, about 7 m/min, about 8 m/min, about 9 m/min, or about 10 m/min.

The at least two of the wound contact layer, the transmission layer and the superabsorbent layer may be laminated by a laminator having a web speed when the laminator is not operating of about 5 m/min, about 10 m/min, about 15 m/min, about 20 m/min, about 25 m/min, about 30 m/min, about 35 m/min, about 40 m/min, about 45 m/min, or about 50 m/min.

The at least two of the wound contact layer, the transmission layer and the superabsorbent layer may be laminated together by a scatter-coat adhesive. The scatter-coat adhesive may be a hot melt, scatter-coat adhesive.

The scatter-coat adhesive may be polylactic acid, polycaprolactone, polyethylene (LDPE, HDPE), polypropylene, polyester, polyamide, ethylene vinyl acetate or polyurethane. Advantageously, each of these materials are cheap and easily obtained.

The scatter-coat adhesive may have a melt flow index of between about 5.2 g/10min and about 11.3 g/10min, as tested with 2.16 kg, 1" PVC die at 160°C.

The scatter-coat adhesive may have a maximum water content of between about 0.25% and about 0.45%, about 0.30% and about 0.40%, or about 0.35%. The scatter-coat adhesive may have a maximum water content of about 0.25%, about 0.30%, about 0.35%, about 0.40%, or about 0.45%.

The scatter-coat adhesive may comprise particles of which at least 98% have a particle size less than 600µm, as measured by DIN EN ISO 4610.

The scatter-coat adhesive may have a mean molecular weight of between about 35,000 g/mol and about 65,000 g/mol, about 40,000 g/mol and about 60,000 g/mol, about 45,000 g/mol and about 55,000 g/mol, or about 50,000 g/mol.

The scatter-coat adhesive may have a melting point of between about 55°C and about 65°C, or between about 58°C and about 62°C, or about 60°C.

The scatter-coat adhesive may have a solubility parameter of 9.30 cal/cm³ to 9.45 cal/cm³, for example of 9.34 cal/cm³ to 9.43 cal/cm³.

In step c, the at least two of the wound contact layer, transmission layer and the superabsorbent layer may be adhered together by stitchbonding.

In step c, each of the wound contact layer, transmission layer and the superabsorbent layer may be adhered together by stitchbonding.

Prior to step c, the wound contact layer may be stitchbonded using lyocell yarn.

The invention according to the second aspect may of course individually include any one or more of the features, optional or otherwise, of the invention according to any other aspect.

According an embodiment, there is provided a negative pressure wound exudate management system comprising the wound dressing of the first aspect, and further comprising:
a source of negative pressure; and
a conduit or tube for providing negative pressure to the wound dressing via an internal lumen of the conduit or tube and an opening in the wound dressing.

The source of negative pressure may comprise a pump for generating negative pressure and the tube connects the pump and wound dressing.

The source of negative pressure may further comprise a one-way valve in-line between the pump and the wound dressing to maintain a negative pressure within the wound dressing when the pump is disconnected from the tube. Normal operation of the pump may include the generation of negative pressure, alternating periods of negative pressure generation, and/or no generation of negative pressure.

The one-way valve may be connected to one of the pump and the tube.

The opening in the wound dressing may be in a backing layer of the wound dressing. The backing layer of the wound dressing may be a bacterial and viral barrier layer which preferably resists the ingress of liquid and air but allows moisture vapour transmission. In this way the backing layer enhances the overall fluid handling capacity of the wound dressing by allowing for the escape of moisture vapour through the backing layer while enabling the application of negative pressure to the wound. The backing layer may be a layer having a Moisture Vapor Transmission Rate (MVTR) of at least about 10,000 g/m² per 24 hours or in the range of from about 10,000g/m² to about 50,000g/m² per 24 hours as measured by the method described in BS EN 13726-22002 "Test methods for primary wound dressings Part 2 Moisture vapour transmission rate of permeable film dressings". The backing layer may be in the form of a film of polyurethane.

The conduit or tube may comprise a proximal end having an opening. The proximal end of the conduit or tube may be adhered around the opening in the backing layer of the wound dressing such that the opening in the backing layer is in fluid communication with the opening in the proximal end of the conduit or tube. The adhesion between the proximal end of the conduit or tube and the opening in the backing layer may for an airtight, or substantially airtight, seal between the conduit or tube and the backing layer.

The backing layer may be provided with a port, or airway for connection to the conduit. The port or airway may be located in that part of the backing layer that overlies the absorbent structure but towards the periphery of the absorbent structure so that it is not directly in vertical alignment with the centre of the wound dressing (or the wound when in use). This assists in the spread of wound exudate across the full extent of the absorbent layer.

The invention according to the third aspect may of course individually include any one or more of the features, optional or otherwise, of the invention according to any other aspect.

### Detailed Description of the Invention

In order that the invention may be more clearly understood one or more embodiments thereof will now be described, by way of example only, with reference to the accompanying drawings, of which:
- Figure 1: is an example embodiment of part of an adhesive skin contact layer for use with a negative pressure wound dressing;
- Figure 2: is an expanded view of section A of Figure 1;
- Figure 3: is an exploded view of a first embodiment of a negative pressure wound dressing;
- Figure 4: is a plan view of a further example embodiment of a negative pressure wound dressing;
- Figure 5: is an exploded view of the negative pressure wound dressing of Fig. 4;

- Figure 6: is a plan view of an example embodiment of a backing layer for use with the embodiment shown in Fig. 4;
- Figure 7: is a perspective view of the example backing layer shown in Fig. 6;
- Figure 8: is a plan view of an example embodiment of an absorbent structure for use with the embodiment shown in Fig. 4;
- Figure 9: is a side view of the example absorbent structure shown in Fig. 8;
- Figure 10: is a rear view of the example absorbent structure shown in Fig. 8;
- Figure 11: is a cross-section view along line A-A shown in Fig. 8;
- Figure 12: is a perspective view of the example absorbent structure shown in Fig. 8;
- Figure 13: is a plan view of a lower surface of an example embodiment of an adhesive skin contact layer for use with the embodiment shown in Fig. 4;
- Figure 14: is a perspective view of the example adhesive skin contact layer shown in Fig. 13;
- Figure 15: is a further plan view of the example embodiment of the negative pressure wound dressing shown in Fig. 4;
- Figure 16: is a cross-section view along line B-B shown in Fig. 15;
- Figure 17: is a perspective view of the example embodiment of the negative pressure wound dressing shown in Fig. 4; and
- Figure 18: is a perspective view of an example embodiment of a release liner for use with the embodiment shown in Fig. 4.

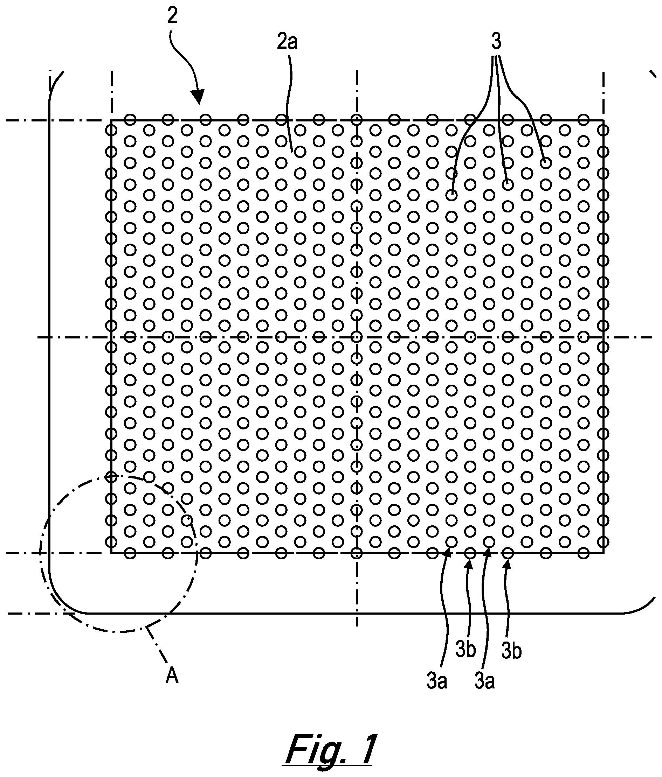

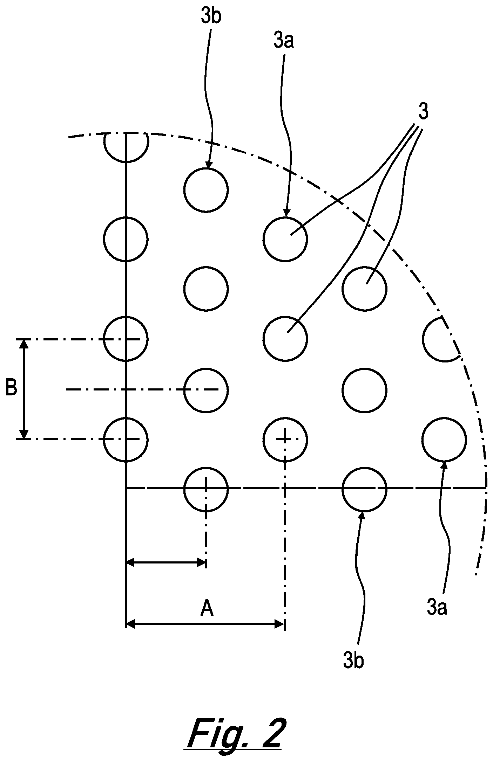

With reference to Figures 1 and 2, there is shown an adhesive skin contact layer 2 for use with a negative pressure wound dressing.

The adhesive skin contact layer assists in securing the wound dressing to the skin of a patient, in particular to the peri-wound skin adjacent to a wound. The adhesive skin contact layer 2 may have a weight of 200gsm which provides for a malleable, gelled structure so, in the event of an air leak, the layer can seal the leak more easily than if the layer had a weight below 100gsm, in particular below 40gsm. The adhesive skin contact layer 2 is a perforated mesh comprising a plurality of perforations 3 arranged in a plurality of rows. In the present embodiment, 95% of the perforations 3 are arranged in a triangle packed layout. The triangle packed layout comprises adjacent rows 3a, 3b of perforations 3. Row 3a is arranged offset from row 3b. The perforations 3 in row 3a are not arranged adjacent to the perforations 3 in the adjacent row 3b but instead the perforations 3 in row 3a are arranged adjacent to the gap between perforations 3 in row 3b or adjacent to part of the gap and part of a perforation 3 in row 3b.

Each perforation 3 comprises an opening, which in this embodiment is in the shape of a circle. In this embodiment, the size of each perforation 3 is defined by the diameter of the circle. However, the person skilled in the art will understand that the size of the opening may be defined depending on the shape of the opening. In some embodiments, the size of a circle is the diameter. In other embodiments, the size of an oval is the longer diameter. In further embodiments, the size of a hexagon is the longest diagonal; and the size of a triangle, a square, a rectangle, an octagon or any other polygon perforation may be the longest side. In the present embodiment, each perforation 3 has a diameter of 2.20mm and are therefore of sufficient size to provide breathability to the wound dressing in the event that some of the perforations reduce in diameter after their formation, for example by the adhesive entering the opening of a perforation. As such, the adhesive skin contact layer maintains an environment optimised for wound healing. Moreover, each perforation 3 is a through hole in the adhesive skin contact layer 2 which can enable fluid to flow through the layer 2. The adhesive skin contact layer 2, therefore, helps to prevent tissue ingrowth into the other material of the wound dressing.

The perforations 3 have a pitch (i.e., the distance between the centre point of a perforation and the centre point of an adjacent perforation) in a first direction (shown as 'A' in Figure 2) of 8.0mm, and a pitch in a second direction (shown as 'B' in Figure 2) of 5.6mm. Advantageously, the pitch of the perforations 3 has a ratio of perforation : no-perforation which provides efficient moisture evaporation, prevents pooling and strongly adheres to the peri-wound skin of the patient. Surprisingly, and advantageously, tests have shown that the adhesive skin contact layer 2 remains adhered to a patient for at least seven days.

The total area of the perforations 3 may be 2100mm² and the total volume of the perforations may be 2100mm³. The degree of perforation may be 10.66% of the area of the adhesive skin contact layer 2. The perforations 3 are distributed evenly throughout the adhesive skin contact layer 2. The perforations 3 may be formed by a mechanical punch perforating process which does not result in the formation of a doughnut or volcano structure around the perforations after formation of the perforations 3. The perforations may be formed by mechanical punch perforating the adhesive skin contact layer 2 comprising a release liner (not shown) covering at least one surface of the adhesive skin contact layer 2.

The adhesive skin contact layer 2 has a thickness of 1.0mm and a width and length (measured from the periphery of the adhesive skin contact layer 2) each of 130mm. The adhesive skin contact layer 2 has an outer periphery in the shape of a square. The adhesive skin contact layer 2 comprises an upper surface 2a and a lower surface 2b. The lower surface 2b contacts the dermal surface in use.

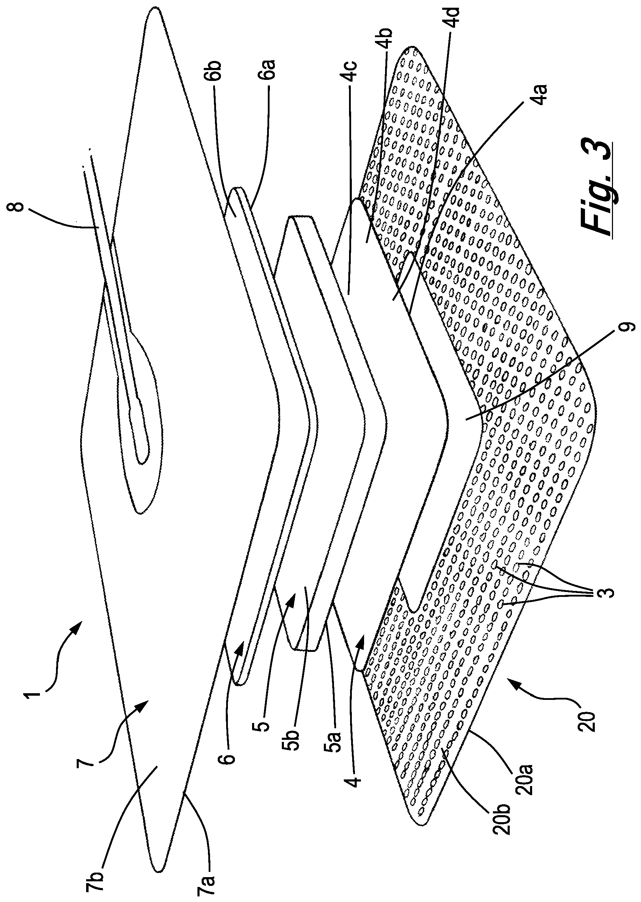

With reference to Figure 3, there is shown a negative pressure wound dressing 1.

The wound dressing 1 comprises an adhesive skin contact layer 20 (which is preferably the adhesive skin contact layer 2 described with reference to Figures 1 and 2), a wound contact layer 4, a transmission layer 5, a superabsorbent layer 6, a backing layer 7 and an airway 8.

The adhesive skin contact layer 20 may have a perimetral shape with a central window 9, therefore, the adhesive skin contact layer 20 may be an adhesive skin contact border layer 20. The border layer 20 may comprise an outer periphery which defines the outer shape of the border layer 20, which, in this embodiment, is a square. The border layer 20 may comprise an inner periphery which defines the shape of the window 9. The window 9 may be of the same, or similar shape, to that of the outer periphery of the border layer 20.

The window 9 is a square through hole in the adhesive skin contact layer 20. The window 9 is located centrally relative to the periphery of the adhesive skin contact layer 20. Each side of the window 9 is arranged parallel to each of the corresponding sides of the adhesive skin contact layer 20. The window may have an area of 12,500mm2. The skilled person will appreciate that the window 9 may be enlarged by any suitable means, for example by cutting the adhesive skin contact layer 20. The window 9 in the adhesive skin contact layer 20 enables the wound of a patient to contact, or be in fluid communication with, other layers of the wound dressing 1, for example the wound contact layer 4, when the wound dressing 1 is applied to the skin of a patient.

The adhesive skin contact layer 20 comprises an upper surface 20b and a lower surface 20a. The lower surface 20a contacts the dermal surface in use. **In** use, the lower surface 20a of the adhesive skin contact layer 20 is detachably adhered to a dermal surface (not shown) of a patient such that the wound site (not shown) is located within the window 9. As such, the adhesive skin contact layer 20 is not in direct contact with the wound site but does wholly surround it. Thus, the adhesive skin contact layer 20 is in contact with intact peri-wound skin only. The upper surface 20b may be outwardly facing away from the patient's body.

A removable cover or "release layer" (not shown) may be adhered to the lower surface 20a of the adhesive skin contact layer 20. The removable cover, which may comprise folded grip sections, is removable from the lower surface 20a of the adhesive skin contact layer 20. Thus, the removable cover protects the adhesive skin contact layer 20 when the removable cover is adhered to the adhesive skin contact layer 20, but when the removable cover is removed from the lower surface 20a of the adhesive skin contact layer 20 for use of the wound dressing 1, the lower surface 20a is exposed and able to releasably secure the wound dressing 1 to the skin of a patient.

The wound contact layer 4, the transmission layer 5 and the superabsorbent layer 6 form an absorbent structure arranged between the adhesive skin contact layer 20 and the backing layer 7.

The backing layer 7 may comprise a first surface 7a and a second surface 7b. In use, the first surface 7a may be inwardly facing toward the patient's body, and the second surface 7b may be outwardly facing away from the patient's body.

The absorbent structure 4, 5, 6 may comprise a first surface 4a and a second surface 6b. The first surface 4a of the absorbent structure 4, 5, 6 may be adjacent, and in contact with, the upper surface 20b of the adhesive skin contact layer 20. The peripheral edge of the second surface 6b of the absorbent structure 4, 5, 6 may be adjacent, and in contact with, the first surface 7a of the backing layer 7.

The wound contact layer 4 may comprise a first surface 4a and a second surface 4b. In use, the first surface 4a may be inwardly facing toward the patient's body, and the second surface 4b may be outwardly facing away from the patient's body. The first surface 4a of the wound contact layer 4 may contact the wound when the wound dressing 1 is adhered to the skin adjacent to the wound (i.e., when in use). The first surface 4a of the wound contact layer 4 is substantially aligned with the window 9 of the adhesive skin contact layer 20. As such, in use, the first surface 4a contacts the wound through the window 9. The second surface 4b of the wound contact layer 4 may be adjacent, and in contact with, a first surface 5a of the transmission layer 5 or a first surface 6a of the superabsorbent layer 6. In the described embodiment, the second surface 4b of the wound contact layer 4 is adjacent, and in contact with, the first surface 5a of the transmission layer 5.

The transmission layer 5 may comprise a first surface 5a and a second surface 5b. In use, the first surface 5a may be inwardly facing toward the patient's body, and the second surface 5b may be outwardly facing away from the patient's body. The first surface 5a of the transmission layer 5 may be adjacent, and in contact with, the second surface 4b of the wound contact layer 4 or a second surface 6b of the superabsorbent layer 6. The second surface 5b of the transmission layer 5 may be adjacent, and in contact with, a first surface 6a of the superabsorbent layer 6 or the first surface 7a of the backing layer 7. In the described embodiment, the first surface 5a of the transmission layer 5 is adjacent, and in contact with, the second surface 4b of the wound contact layer, and the second surface 5b of the transmission layer 5 is adjacent, and in contact with, the first surface 6a of the superabsorbent layer 6.

The superabsorbent layer 6 may comprise a first surface 6a and a second surface 6b. In use, the first surface 6a may be inwardly facing toward the patient's body, and the second surface 6b may be outwardly facing away from the patient's body. The first surface 6a of the superabsorbent layer 6 may be adjacent, and in contact with, the second surface 4b of the wound contact layer 4 or the second surface 5b of the transmission layer 5. The second surface 6b of the superabsorbent layer 6 may be adjacent, and in contact with, the first surface 5a of the transmission layer 5 or the first surface 7a of the backing layer 7. In the described embodiment, the first surface 6a of the superabsorbent layer 6 is adjacent, and in contact with, the second surface 5b of the transmission layer 5, and the second surface 6b of the superabsorbent layer 6 is adjacent, and in contact with, the first surface 7a of the backing layer 7.

The adhesive skin contact layer 20 may radially overlap the absorbent structure 4, 5, 6. The adhesive skin contact layer 20 may comprise an interior portion adjacent to and surrounding the window 9, and an exterior portion radially outwardly from the interior portion. The interior portion and the exterior portion of the first surface 20a of the adhesive skin contact layer 20 may contact and adhere to the skin of a patient in use. The interior portion of the second surface 20b of the adhesive skin contact layer 20 may be adjacent, and in contact with, a portion of the first surface 4a of the wound contact layer 4. The exterior portion of the second surface 20b of the adhesive skin contact layer 20 may be adjacent, and joined with, a peripheral portion of the first surface 7a of the backing layer 7. Beneficially, this helps to prevent wound exudate escaping from the confines of the wound dressing which would be unhygienic and cause discomfort for the user. Moreover, this also helps maintain a negative pressure at the wound site.

The exterior portion of the second surface 20b of the adhesive skin contact layer 20 may be joined to the peripheral portion of the first surface 7a of the backing layer 7 by heat lamination, adhesive, welding or stitching.

The exterior portion of the second surface 20b of the adhesive skin contact layer 20 may extend radially beyond the periphery of the absorbent structure by about 27.5 mm.

At least two of the wound contact layer 4, the transmission layer 5 and the superabsorbent layer 6 may be laminated together. In the described embodiment, each of the wound contact layer 4, the transmission layer 5 and the superabsorbent layer 6 are laminated together. Advantageously, this means that the absorbent structure of the present invention is manufactured without requiring any relatively rigid dressing and binding components. Thus, the present invention provides an absorbent structure which is flexible, therefore benefits user comfort, and which is cheaper to manufacture than absorbent structures and, therefore negative pressure wound dressings, of the prior art. Moreover, lamination does not require any complex equipment or manufacturing techniques. Thus, the present invention is simple to manufacture.

Each of the wound contact layer 4, the transmission layer 5 and the superabsorbent layer 6 may be laminated together by a scatter-coat adhesive. The scatter-coat adhesive may be a hot melt, scatter-coat adhesive. **In** the described embodiment, the scatter-coat adhesive is polycaprolactone and has the following properties:
- a melt flow index of between 5.2 g/10min and 11.3 g/10min, as tested with 2.16 kg, 1" PVC die at 160°C;
- a maximum water content of 0.35%;
- a mean molecular weight of 50,000;
- a melting point of 60°C; and
- a solubility parameter of 9.34 cal/cm3 to 9.43 cal/cm3.

The scatter-coat adhesive may comprise particles of which at least 98% have a particle size of 0.6mm.

The wound contact layer 4, the transmission layer 5 and the superabsorbent layer 6 may be laminated together by a laminator having the following processing properties:
- Heating zone temperature of 125°C;
- Cooling zone temperature of 25°C;
- Web tension (for each unwind reel) of 125 N;
- Crush roller pressure of 1000 N; and
- Web speed of 10 m/min.

The wound contact layer 4 further comprises a central region 4c and a peripheral region 4d. The central region 4c is substantially square and is bordered on each side by the peripheral region 4d.

In the described embodiment, the first surface 4a, which contacts the wound in use, may comprise warp stitches (longitudinal to the plane of the wound contact layer 4) only, i.e., the first surface 4a does not comprise any weft stitches (transverse to the plane of the wound contact layer 4). Advantageously, this means that the amount of non-gelling fibres (which may include weft stitches) at the wound contact surface is minimised. Thus, there is a greater ratio of gelling fibres to non-gelling fibres at the wound contact surface in contact with the wound and, therefore, wound exudate. Thus, the absorption capacity of the wound contact layer is increased. As such, pooling of wound exudate at the wound site is prevented, or at least significantly reduced and an environment optimised for wound healing is maintained at the wound site.

The warp stitches of the first surface 4a may have a pitch of 6.6mm and a stitch density of 7.5 stitches /cm.

The second surface 4b, which is laminated to the first surface 5a of the transmission layer 5, comprises warp stitches and weft stitches. Each of the warp stitches and the weft stitches of the second surface 4b have a pitch of 6.6mm and a stitch density of 7.5 stitches /cm.

In other embodiments, the central region 4c of the first surface 4a may comprise warp stitches only and the peripheral region 4d of the first surface 4a may comprise warp and weft stitches.

The wound contact layer 4 may have a weight of 100gsm and a thickness of 3.0 mm.

Between the first surface 4a and the second surface 4b, which are joined about their periphery, the wound contact layer 4 may comprise a plurality of gel-forming fibres which, upon the uptake of wound exudate, become moist, slippery or gelatinous. Specifically, in this embodiment, the gel-forming fibres are hygroscopic fibres which upon the uptake of wound exudate become gelatinous. The gel-forming fibres may be of the type which retain their structural integrity on absorption of exudate. In the present embodiment, the fibres are sodium carboxymethylcellulose fibres having a degree of substitution of 0.30 carboxymethyl groups per glucose unit, as measured by IR spectroscopy (as defined in WO00/01425). The absence of weft stitches at the first surface 4a means that a greater amount of gel-forming fibres, as described above, are exposed at the first surface and, therefore, in contact with the wound. As such, the wound contact layer is higher gelling, i.e., has a significantly greater absorbency capacity and is more retentive, than gel-forming fibres of the prior art. As such, the wound contact layer 4 absorbs a greater amount of wound exudate. Thus, pooling of wound exudate at the wound site is prevented, or at least significantly reduced.

The sodium carboxymethylcellulose fibres are made by carboxymethylating a non-woven cellulosic fabric having an absorbency of 18g/g of Solution A (sodium/calcium chloride solution), as measured by the method described in BS EN 13726-1 (2002) "Test methods for primary wound dressings", section 3.2 "Free swell absorptive capacity". The carboxymethylation is performed by contacting the fabric with an alkali and a carboxymethylating agent such as chloroacetic acid in a combined alcohol and aqueous system. The cellulosic fabric comprises solely of cellulosic fibre and comprises continuous filament yarn.

The gel-forming fibres have an absorbency of 2 grams 0.9% saline solution per gram of fibre (as measured by the free swell method).

The wound contact layer 4 is stitched using lyocell yarn (produced under the trade name Tencel^{™} yarn).

The transmission layer 5 is substantially square, as is the wound contact layer 4 and the superabsorbent layer 6, and has a thickness of 1.8mm and is made of a polyurethane foam. The transmission layer 5 may have a density of between 26 Kg/M3 and 28 Kg/M3 and a tensile strength of 150 kPa. The transmission layer 5 may have an elongation at break value of 300% and a cell count of between 17 and 26 /cm. Moreover, the transmission layer 5 may have a 40% CLD hardness of between 2.5 and 4.5 kPa.

The superabsorbent layer 6 comprises an upper layer having the second surface 6b, and a lower layer having the first surface 6a. Between the upper layer and the lower layer, there is enclosed a first material comprising non-woven fibres, an absorbent material and a hot melt binder.

The hot melt binder may be present in an amount of 70 g/m². The hot melt binder may be a copolymer and, in the described embodiment, may be ethylene-vinyl acetate (EVA). In manufacture of the superabsorbent layer, the superabsorbent layer may be heated to melt the hot melt binder and bind the non-woven fibres of the first material together. Advantageously, this prevents, or at least significantly limits, the amount of shedding of the fibres of the superabsorbent layer when in use. Thus, the presence of the hot melt binder in the superabsorbent layer significantly increases the strength of the layer, preventing dissociation of the absorbent structure.

The absorbent material may be a superabsorbent material, capable of absorbing wound exudate while allowing the passage of fluid through it. The superabsorbent material may be sodium polyacrylate and in the form of a powder which, advantageously, increases the surface area to volume ratio of the superabsorbent material, therefore enhancing the absorbent property of the material.

The non-woven fibres may be cellulose fibres. The cellulose non-woven fibres may be arranged as a cellulosic non-woven matrix of fibres and may be a support onto which the absorbent material may be incorporated, for example, in the described embodiment, the absorbent material is dispersed upon the cellulosic non-woven matrix. The absorbent material may be dispersed evenly, or substantially evenly, throughout or upon the non-woven fibres, for example the cellulosic non-woven matrix.

The upper layer and the lower layer of the superabsorbent layer 6 may each be a cellulosic fabric layer. The upper fabric layer and the lower fabric layer may be joined around their entire periphery to enclose the first material.

The superabsorbent layer 6 may comprise a plurality of fenestrations (not shown). Each of the plurality of fenestrations may be openings in the form of slits in the superabsorbent layer 6. Each fenestration may have a length equal to 80% of the length of the superabsorbent layer 6. Each of the upper layer and the lower layer may comprise a plurality of fenestrations. The fenestrations may be arranged in a direction planar to a longitudinal axis and transverse to a longitudinal axis of the superabsorbent layer 6. Advantageously, this arrangement aids in managing a flow of exudate through the superabsorbent layer 6 of the wound dressing 1. The fenestrations encourage or enable wound exudate to travel laterally on the superabsorbent layer 6, as opposed to axially, and assist in negative pressure transmission through the superabsorbent layer 6.

The superabsorbent layer 6 may have a basis weight of 460 g/m2, a thickness of 2.55 mm and a density of 0.255 g/cm3. Moreover, the superabsorbent layer 6 may have a tensile strength (dry) of 45 N/50mm, an absorbent capacity of 25 g/g 10min, and an absorbency under compression capacity of 0.85 g/cm2.

Each of the wound contact layer 4, transmission layer 5 and superabsorbent layer 6 are substantially planar.

In use, the wound dressing 1 forms part of a negative pressure wound exudate management system comprising the wound dressing 1 and further comprising a source of negative pressure (not shown) and a coupling member (for example, airway 8) for providing negative pressure to the wound dressing from an internal lumen of a conduit or tube (not shown) and through an opening (not shown) in the wound dressing. The source of negative pressure comprises a pump for generating negative pressure and the tube connects the pump and wound dressing 1 via the airway 8. In the present embodiment, the source of negative pressure may comprise a one-way valve (not shown) in-line between the pump and the wound dressing 1 to maintain a negative pressure within the wound dressing 1 when the pump is disconnected from the tube.

In use, the adhesive skin contact layer 20 is adhered to the intact peri-wound skin around a wound. The wound is located within the window 9 of the adhesive skin contact layer 20. The wound dressing 1 is connected to a source of negative pressure before or after application of the dressing to the wound. Upon leaving the wound, wound exudate contacts the first surface 4a of the wound contact layer 4 and wicks upwardly, away from the wound site, toward the transmission layer 5. The high gelling ability of the wound contact layer 4 facilitates absorption and retention of the wound exudate. Lamination of the wound contact layer 4, transmission layer 5 and superabsorbent layer 6 means each of these layers form a consolidated, absorbent and retentive structure. The wound exudate wicks from the upper surface 4b of the wound contact layer 4 to the transmission layer 5. Lateral movement of the exudate occurs in the transmission layer 5 to spread the wound exudate across the surface area of the dressing. The exudate then wicks upwardly to the superabsorbent layer 6 where it is absorbed and retained prior to being transmitted out of the dressing 1 via vapour transmission through the backing film 7.

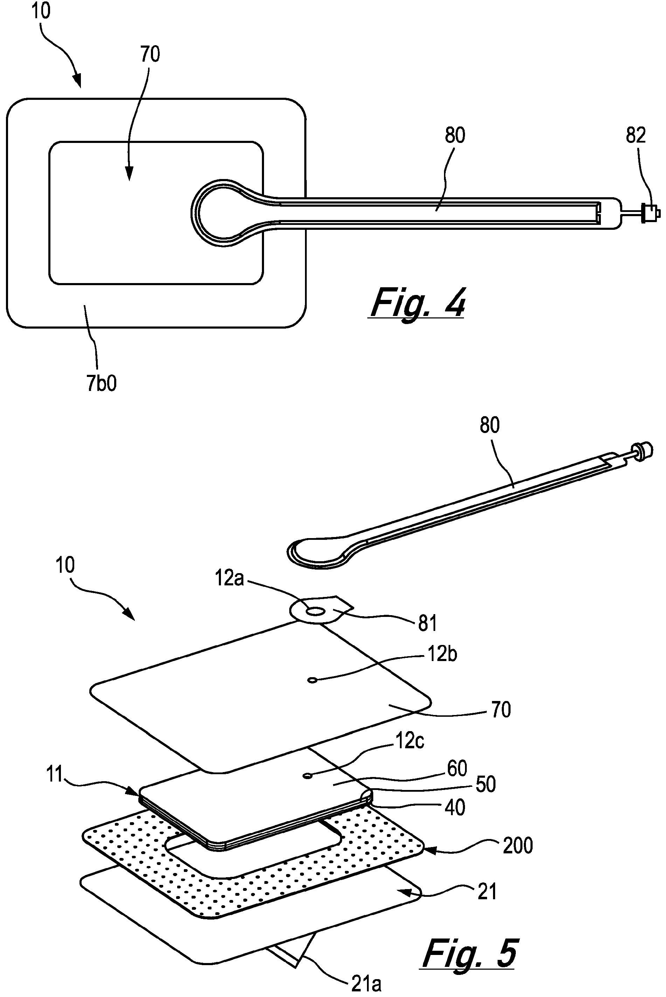

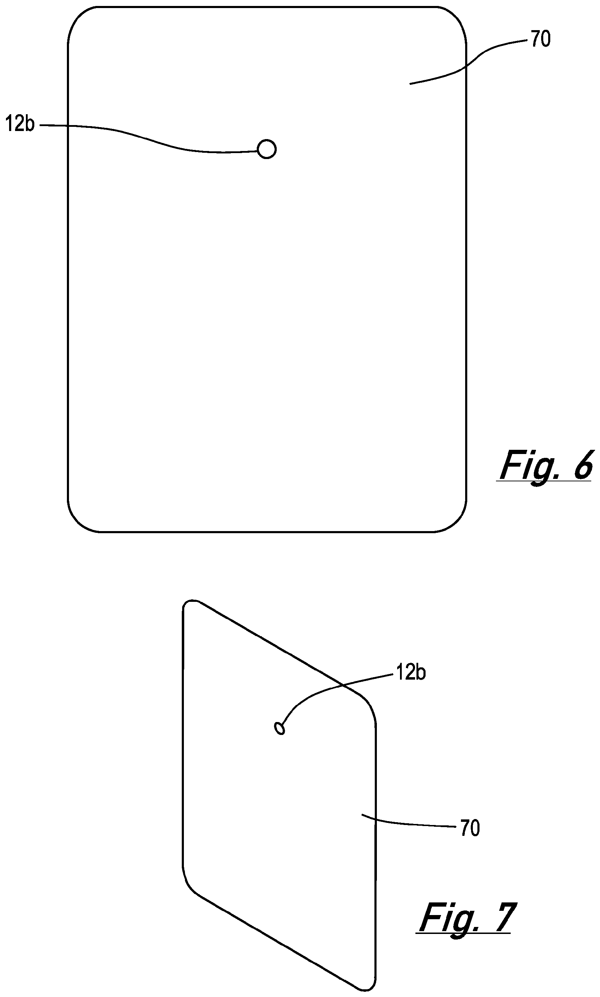

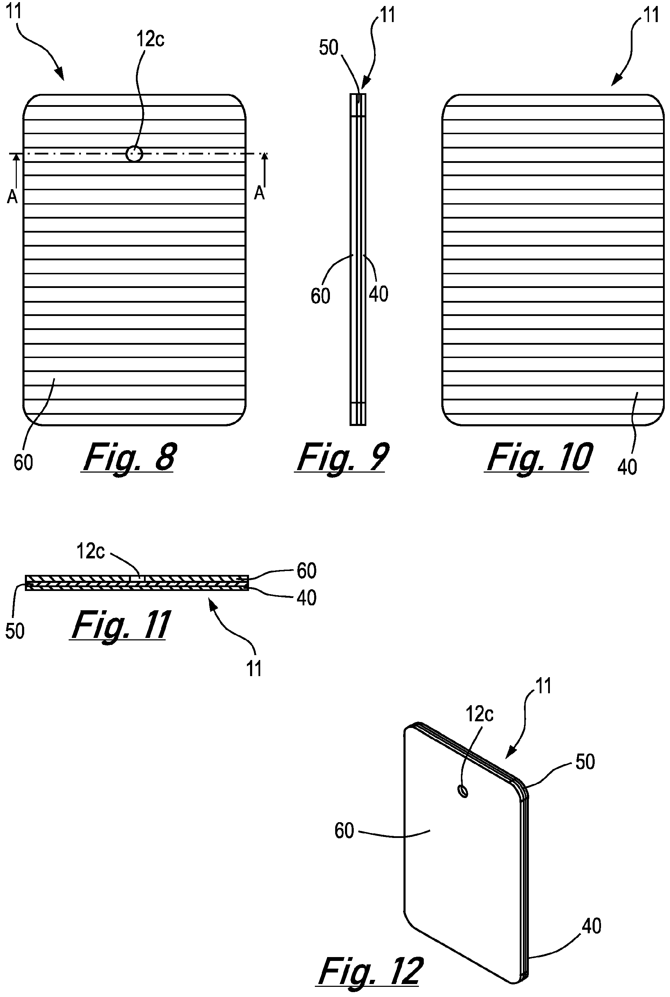

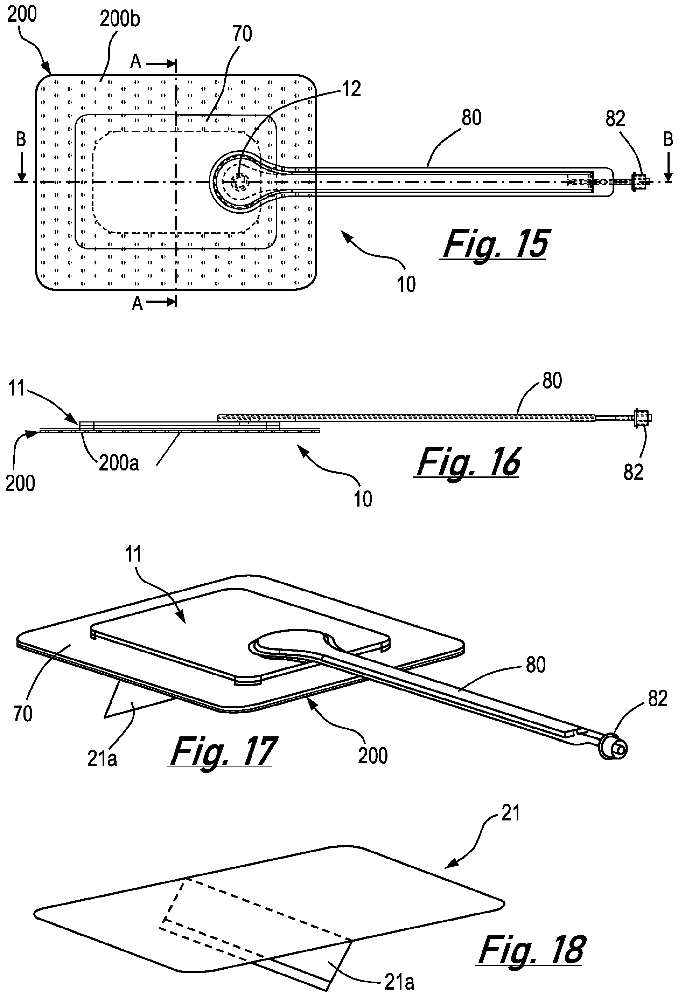

With reference to Figures 4 to 18, there is shown a further example embodiment of a negative pressure wound dressing 10.

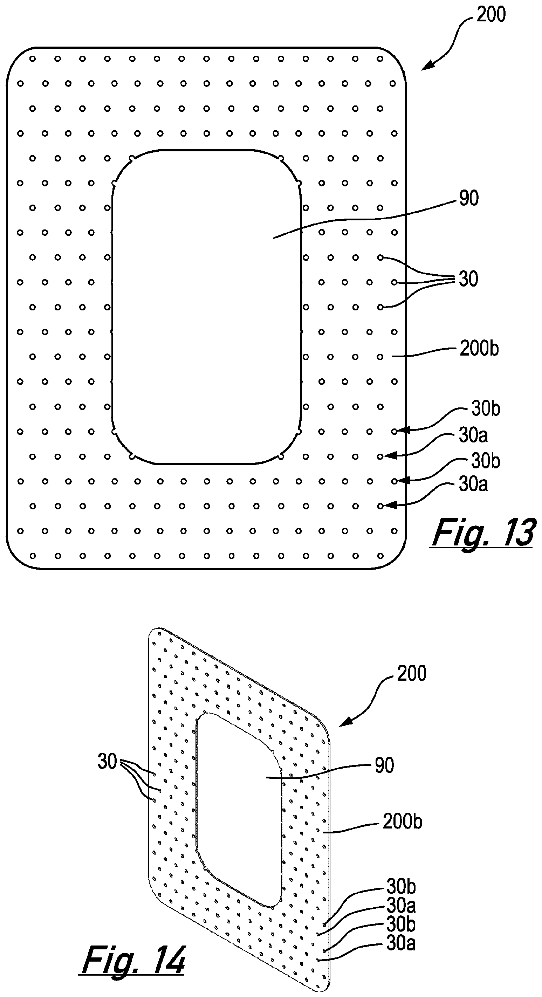

The wound dressing 10 comprises an adhesive skin contact layer 200 (which is preferably the adhesive skin contact layer 200 described with reference to Figures 13 and 14), a wound contact layer 40, a transmission layer 50, a superabsorbent layer 60, a backing layer 70 and an airway 80. The wound contact layer 40, transmission layer 50 and superabsorbent layer 60 together form an absorbent structure 11 arranged between the adhesive skin contact layer 200 and the backing layer 70.

The adhesive skin contact layer 200 assists in securing the wound dressing 10 to the skin of a patient, in particular to the peri-wound skin adjacent to a wound. The adhesive skin contact layer 200 may have a weight of 200gsm which provides for a malleable, gelled structure so, in the event of an air leak, the layer can seal the leak more easily than if the layer had a weight below 100gsm, in particular below 40gsm. With reference to Figs. 13 and 14, the adhesive skin contact layer 200 may be a perforated mesh comprising a plurality of perforations 30 arranged in a plurality of rows. In the present embodiment, 95% of the perforations 30 are arranged in a triangle packed layout. The triangle packed layout comprises adjacent rows 30a, 30b of perforations 30. Row 30a is arranged offset from row 30b. The perforations 30 in row 30a are not arranged adjacent to the perforations 30 in the adjacent row 30b but instead the perforations 30 in row 30a are arranged adjacent to the gap between perforations 30 in row 30b or adjacent to part of the gap and part of a perforation 30 in row 30b.

Each perforation 30 comprises an opening, which in this embodiment is in the shape of a circle. In this embodiment, the size of each perforation 30 is defined by the diameter of the circle. However, the person skilled in the art will understand that the size of the opening may be defined depending on the shape of the opening. In some embodiments, the size of a circle is the diameter. In other embodiments, the size of an oval is the longer diameter. In further embodiments, the size of a hexagon is the longest diagonal; and the size of a triangle, a square, a rectangle, an octagon or any other polygon perforation may be the longest side. In the present embodiment, each perforation 30 has a diameter of 2.20mm and are therefore of sufficient size to provide breathability to the wound dressing in the event that some of the perforations reduce in diameter after their formation, for example by the adhesive entering the opening of a perforation. As such, the adhesive skin contact layer maintains an environment optimised for wound healing. Moreover, each perforation 30 is a through hole in the adhesive skin contact layer 200 which can enable fluid to flow through the layer 200. The adhesive skin contact layer 200, therefore, helps to prevent tissue ingrowth into the other material of the wound dressing.

The perforations 30 may have a pitch (i.e., the distance between the centre point of a perforation and the centre point of an adjacent perforation) in a first direction (shown as 'A' in Figure 2 and applicable to the perforations 30 shown in Figs. 13 and 14) of 8.0mm, and a pitch in a second direction (shown as 'B' in Figure 2 and applicable to the perforations 30 shown in Figs. 13 and 14) of 5.6mm. Advantageously, the pitch of the perforations 30 has a ratio of perforation : no-perforation which provides efficient moisture evaporation, prevents pooling and strongly adheres to the peri-wound skin of the patient. Surprisingly, and advantageously, tests have shown that the adhesive skin contact layer 200 remains adhered to a patient for at least seven days.

The total area of the perforations 30 may be 2100mm² and the total volume of the perforations may be 2100mm³. The degree of perforation may be 10.66% of the area of the adhesive skin contact layer 200. The perforations 30 are distributed evenly throughout the adhesive skin contact layer 200. The perforations 30 may be formed by a mechanical punch perforating process which does not result in the formation of a doughnut or volcano structure around the perforations after formation of the perforations 30. The perforations may be formed by mechanical punch perforating the adhesive skin contact layer 200 comprising a release liner (not shown) covering at least one surface of the adhesive skin contact layer 200.

The adhesive skin contact layer 200 may have a thickness of 1.0mm and a width and length (measured from the periphery of the adhesive skin contact layer 200) each of 130mm. The adhesive skin contact layer 200 has an outer periphery in the shape of a square. The adhesive skin contact layer 200 comprises an upper surface 200a and a lower surface 200b. The lower surface 200b contacts the dermal surface in use.

The adhesive skin contact layer 200 may have a perimetral shape with a central window 90, therefore, the adhesive skin contact layer 200 may be an adhesive skin contact border layer 200. The border layer 200 may comprise an outer periphery which defines the outer shape of the border layer 200, which, in this embodiment, is a rectangle. The border layer 200 may comprise an inner periphery which defines the shape of the window 90. The window 90 may be of the same, or similar shape, to that of the outer periphery of the border layer 200. In other embodiments, the window may have a different to that of the outer periphery of the border layer 200. For example, in embodiments comprising a border layer having an outer periphery which is square, the window may be, for example, a square or a rectangle. In embodiments, comprising a border layer having a square outer periphery and a window which is a rectangle, there may be greater overlap between the absorbent structure and the adhesive skin contact layer, therefore, allowing for a greater surface area for adhesion between the adhesive skin contact layer and the absorbent structure.

In this embodiment, the window 90 has a perimeter which is a rectangle, therefore, the length of the transverse sides of the window is less than the length of the longitudinal sides of the window 90. The window 90 is a rectangle through-hole in the adhesive skin contact layer 200. The shape of the window 90 provides for greater overlap of the absorbent structure 11 and the adhesive skin contact layer 200, especially at the corresponding corner portion of the absorbent structure 11 and the adhesive skin contact layer 200. This is beneficial not least because it allows for a greater surface area for adhesion between the adhesive skin contact layer 200 and the absorbent structure 11, therefore, providing greater structural integrity to the wound dressing 10. The greater structural integrity means that the absorbent structure 11 maintains its position within the wound dressing 10 with reduced possibility of the absorbent structure 11 becoming dislodged from its position within the wound dressing 10 when in use, in particular when in use when the wound dressing 10 is wet with exudate or other fluids.

The window 90 is located centrally relative to the periphery of the adhesive skin contact layer 200. Each side of the window 90 is arranged parallel to each of the corresponding sides of the adhesive skin contact layer 200. Each longitudinal side of the window 90 may have a length of about 210mm and each traverse side of the window 90 may have a length of about 160mm. The window may have an area of about 33,600mm². The skilled person will appreciate that the window 90 may be enlarged by any suitable means, for example by cutting the adhesive skin contact layer 200. The window 90 in the adhesive skin contact layer 200 enables the wound of a patient to contact, or be in fluid communication with, other layers of the wound dressing 10, for example the wound contact layer 40, when the wound dressing 10 is applied to the skin of a patient.

The adhesive skin contact layer 200 comprises an upper surface 200b and a lower surface 200a. The lower surface 200a contacts the dermal surface in use. In use, the lower surface 200a of the adhesive skin contact layer 200 is detachably adhered to a dermal surface (not shown) of a patient such that the wound site (not shown) is located within the window 90. As such, the adhesive skin contact layer 200 is not in direct contact with the wound site but does wholly surround it. Thus, the adhesive skin contact layer 200 is in contact with intact peri-wound skin only. The upper surface 200b may be outwardly facing away from the patient's body.

A removable cover or "release layer" 21 may be adhered to the lower surface 200a of the adhesive skin contact layer 200. The removable cover 21, which may comprise folded grip sections 21a, is removable from the lower surface 200a of the adhesive skin contact layer 200. Thus, the removable cover protects the adhesive skin contact layer 200 when the removable cover 21 is adhered to the adhesive skin contact layer 200, but when the removable cover 21 is removed from the lower surface 200a of the adhesive skin contact layer 200 for use of the wound dressing 10, the lower surface 200a is exposed and able to releasably secure the wound dressing 10 to the skin of a patient.

The airway 80 may comprise a connector 82 at its distal end. The connector 82 may connect the airway 80 to an internal lumen of a conduit or tube (not shown) which in turn is connected to a source of negative pressure. The source of negative pressure may comprise a pump (not shown) for generating negative pressure and the conduit or tube may connect the pump and wound dressing 10 via the airway 80. The pump may provide negative pressure to the wound dressing 10 via the internal lumen of the conduit or tube, along the airway 80, and through an opening 12 in the wound dressing 10. In the present embodiment, the source of negative pressure may comprise a one-way valve (not shown) in-line between the pump and the wound dressing 1 to maintain a negative pressure within the wound dressing 1 when the pump is disconnected from the tube.

The airway may further comprise an adhesive layer 81 at its proximal end for adhering the proximal end of the airway 80 to a second surface 7b0 (outer facing surface) of the backing layer 70. The adhesive layer 81 may comprise an opening 12a for allowing a flow of air from the wound dressing 10 into the airway 80.

The opening 12 may comprise an opening in each of the backing layer 70 and absorbent structure 11. The backing layer 70 may comprise an opening 12b, and the absorbent structure 11 may comprise an opening 12c. Openings 12b and 12c may be arranged off-centre relative to the periphery of the backing layer 70 and the absorbent structure 11, respectively. Opening 12c may comprise corresponding openings in each of the superabsorbent layer 60, transmission layer 50 and wound contact layer 40. Opening 12c may be in fluid communication with the window 90 and, therefore, the wound site. The openings 12a, 12b and 12c may provide a fluid pathway between the wound site and the airway 80. Thus, in use, the openings 12a, 12b and 12c allow for the flow of air from the wound site, through the absorbent structure 11, through the backing layer 70, along the airway 80 and along a tube or conduit (not shown) toward a source of negative pressure (not shown).

The backing layer 70 may comprise a first surface 7a0 and the second surface 7b0. In use, the first surface 7a0 may be inwardly facing toward the patient's body, and the second surface 7b0 may be arranged opposite the first surface 7a0, outwardly facing away from the patient's body.

The absorbent structure 11 may comprise a first surface and a second surface. In use, the first surface of the absorbent structure 11 may predominantly comprise a wound facing surface of the wound contact layer 40. In use, the second surface of the absorbent structure 11 may predominantly comprise an outwardly facing surface of the superabsorbent layer 60. The first surface of the absorbent structure 11 may be adjacent, and in contact with, the upper surface 200b of the adhesive skin contact layer 200. In particular, the peripheral portion of the first surface of the absorbent structure 11 may be in contact with the upper surface 200b of the adhesive skin contact layer 200. The peripheral edge of the second surface of the absorbent structure 11 may be adjacent, and in contact with, the first surface 7a0 of the backing layer 70.

The wound contact layer 40 may comprise a first surface arranged opposite a second surface. In use, the first surface may be inwardly facing toward a patient's body, and the second surface may be outwardly facing, away from the patient's body. The first surface of the wound contact layer 40 may contact the wound when the wound dressing 10 is adhered to the skin adjacent to the wound (i.e., when in use). The first surface of the wound contact layer 40 may be substantially aligned with the window 90 of the adhesive skin contact layer 200. As such, in use, the first surface contacts the wound through the window 90. The second surface of the wound contact layer 40 may be adjacent, and in contact with, a first surface of the transmission layer 50 or a first surface of the superabsorbent layer 60. In the described embodiment, the second surface of the wound contact layer 40 is adjacent, and in contact with, the first surface of the transmission layer 50.

The transmission layer 50 may comprise a first surface arranged opposite a second surface. In use, the first surface may be inwardly facing toward a patient's body, and the second surface may be outwardly facing away from the patient's body. The first surface of the transmission layer 50 may be adjacent, and in contact with, the second surface of the wound contact layer 40 or a second surface of the superabsorbent layer 60. The second surface of the transmission layer 50 may be adjacent, and in contact with, a first surface of the superabsorbent layer 60 or the first surface 7a of the backing layer 70. In the described embodiment, the first surface of the transmission layer 50 is adjacent, and in contact with, the second surface of the wound contact layer 40, and the second surface of the transmission layer 50 is adjacent, and in contact with, the first surface of the superabsorbent layer 60.

The superabsorbent layer 60 may comprise a first surface arranged opposite a second surface. In use, the first surface may be inwardly facing toward a patient's body, and the second surface may be outwardly facing away from the patient's body. The first surface of the superabsorbent layer 60 may be adjacent, and in contact with, the second surface of the wound contact layer 40 or the second surface of the transmission layer 50. The second surface of the superabsorbent layer 60 may be adjacent, and in contact with, the first surface of the transmission layer 50 or the first surface 7a of the backing layer 70. In the described embodiment, the first surface of the superabsorbent layer 60 is adjacent, and in contact with, the second surface of the transmission layer 50, and the second surface of the superabsorbent layer 60 is adjacent, and in contact with, the first surface 7a of the backing layer 70.

The adhesive skin contact layer 200 may radially overlap the absorbent structure 11. The adhesive skin contact layer 200 may comprise an interior portion adjacent to and surrounding the window 90, and an exterior portion radially outwardly from the interior portion. The interior portion and the exterior portion of the first surface 20a of the adhesive skin contact layer 200 may contact and adhere to the skin of a patient in use. The interior portion of the second surface 200b of the adhesive skin contact layer 200 may be adjacent, and in contact with, a portion of the first surface of the wound contact layer 40. The exterior portion of the second surface 200b of the adhesive skin contact layer 200 may be adjacent, and joined with, a peripheral portion of the first surface 7a0 of the backing layer 70. Beneficially, this helps to prevent wound exudate escaping from the confines of the wound dressing which would be unhygienic and cause discomfort for the user. Moreover, this also helps maintain a negative pressure at the wound site.

The exterior portion of the second surface 200b of the adhesive skin contact layer 200 may be joined to the peripheral portion of the first surface 7a0 of the backing layer 70 by heat lamination, adhesive, welding or stitching.

The exterior portion of the second surface 200b of the adhesive skin contact layer 200 may extend radially beyond the periphery of the absorbent structure by about 27.5 mm.

At least two of the wound contact layer 40, the transmission layer 50 and the superabsorbent layer 60 may be laminated together. In the described embodiment, each of the wound contact layer 40, the transmission layer 50 and the superabsorbent layer 60 are laminated together, therefore forming a consolidated absorbent structure 11. Advantageously, this means that the absorbent structure 11 of the present invention is manufactured without requiring any relatively rigid dressing and binding components. Thus, the present invention provides an absorbent structure 11 which is flexible, therefore benefits user comfort, and which is cheaper to manufacture than absorbent structures and, therefore negative pressure wound dressings, of the prior art. Moreover, beneficially lamination does not require any complex equipment or manufacturing techniques. Thus, the present invention is simple to manufacture.

Each of the wound contact layer 40, the transmission layer 50 and the superabsorbent layer 60 may be laminated together by a scatter-coat adhesive. The scatter-coat adhesive may be a hot melt, scatter-coat adhesive. In the described embodiment, the scatter-coat adhesive is polycaprolactone and has the following properties:
- a melt flow index of between 5.2 g/10min and 11.3 g/10min, as tested with 2.16 kg, 1" PVC die at 160°C;
- a maximum water content of 0.35%;
- a mean molecular weight of 50,000;
- a melting point of 60°C; and
- a solubility parameter of 9.34 cal/cm³ to 9.43 cal/cm³.

The scatter-coat adhesive may comprise particles of which at least 98% have a particle size of 0.6mm.

The wound contact layer 40, the transmission layer 50 and the superabsorbent layer 60 may be laminated together by a laminator having the following processing properties:
- Heating zone temperature of 125°C;
- Cooling zone temperature of 25°C;
- Web tension (for each unwind reel) of 125 N;
- Crush roller pressure of 1000 N; and
- Web speed of 10 m/min.

The wound contact layer 40 may further comprise a central region and a peripheral region. The central region may be substantially square and is bordered on each side by the peripheral region.

In the described embodiment, the first surface, which contacts the wound in use, of the wound contact layer 40 may comprise warp stitches (longitudinal to the plane of the wound contact layer 40) only, i.e., the first surface does not comprise any weft stitches (transverse to the plane of the wound contact layer 40). Advantageously, this means that the amount of non-gelling fibres (which may include weft stitches) at the wound contact surface is minimised. Thus, there is a greater ratio of gelling fibres to non-gelling fibres at the wound contact surface in contact with the wound and, therefore, wound exudate. Thus, the absorption capacity of the wound contact layer is increased. As such, pooling of wound exudate at the wound site is prevented, or at least significantly reduced and an environment optimised for wound healing is maintained at the wound site.

The warp stitches of the first surface may have a pitch of 6.6mm and a stitch density of 7.5 stitches /cm.

The second surface of the transmission layer 50, which is laminated to the first surface of the transmission layer 50, comprises warp stitches and weft stitches. Each of the warp stitches and the weft stitches of the second surface of the transmission layer have a pitch of 6.6mm and a stitch density of 7.5 stitches /cm.

In other embodiments, the central region of the first surface of the wound contact layer 40 may comprise warp stitches only and the peripheral region of the first surface of the wound contact layer 40 may comprise warp and weft stitches.

The wound contact layer 40 may have a weight of 100gsm and a thickness of 3.0 mm.

Between the first surface of the wound contact layer 40 and the second surface pf the wound contact layer 40, which are joined about their periphery, the wound contact layer 40 may comprise a plurality of gel-forming fibres which, upon the uptake of wound exudate, become moist, slippery or gelatinous. Specifically, in this embodiment, the gel-forming fibres are hygroscopic fibres which upon the uptake of wound exudate become gelatinous. The gel-forming fibres may be of the type which retain their structural integrity on absorption of exudate. In the present embodiment, the fibres are sodium carboxymethylcellulose fibres having a degree of substitution of 0.30 carboxymethyl groups per glucose unit, as measured by IR spectroscopy (as defined in WO00/01425). The absence of weft stitches at the first surface of the wound contact layer 40 means that a greater amount of gel-forming fibres, as described above, are exposed at the first surface and, therefore, in contact with the wound. As such, the wound contact layer 40 is higher gelling, i.e., has a significantly greater absorbency capacity and is more retentive, than gel-forming fibres of the prior art. As such, the wound contact layer 40 absorbs a greater amount of wound exudate. Thus, pooling of wound exudate at the wound site is prevented, or at least significantly reduced.

The sodium carboxymethylcellulose fibres may be made by carboxymethylating a non-woven cellulosic fabric having an absorbency of 18g/g of Solution A (sodium/calcium chloride solution), as measured by the method described in BS EN 13726-1 (2002) "Test methods for primary wound dressings", section 3.2 "Free swell absorptive capacity". The carboxymethylation is performed by contacting the fabric with an alkali and a carboxymethylating agent such as chloroacetic acid in a combined alcohol and aqueous system. The cellulosic fabric comprises solely of cellulosic fibre and comprises continuous filament yarn.

The gel-forming fibres may have an absorbency of 2 grams 0.9% saline solution per gram of fibre (as measured by the free swell method).

The wound contact layer 40 may be stitched using lyocell yarn (produced under the trade name TencelTM yarn).

The transmission layer 50 is substantially square, as is the wound contact layer 40 and the superabsorbent layer 60, and has a thickness of 1.8mm and is made of a polyurethane foam. The transmission layer 50 may have a density of between 26 Kg/M³ and 28 Kg/M³ and a tensile strength of 150 kPa. The transmission layer 50 may have an elongation at break value of 300% and a cell count of between 17 and 26 /cm. Moreover, the transmission layer 50 may have a 40% CLD hardness of between 2.5 and 4.5 kPa.

The superabsorbent layer 60 comprises an upper layer having the second surface, and a lower layer having the first surface. Between the upper layer and the lower layer, there is enclosed a first material comprising non-woven fibres, an absorbent material and a hot melt binder.

The hot melt binder may be present in an amount of 70 g/m². The hot melt binder may be a copolymer and, in the described embodiment, may be ethylene-vinyl acetate (EVA). In manufacture of the superabsorbent layer, the superabsorbent layer may be heated to melt the hot melt binder and bind the non-woven fibres of the first material together. Advantageously, this prevents, or at least significantly limits, the amount of shedding of the fibres of the superabsorbent layer when in use. Thus, the presence of the hot melt binder in the superabsorbent layer significantly increases the strength of the layer, preventing dissociation of the absorbent structure.

The absorbent material may be a superabsorbent material, capable of absorbing wound exudate while allowing the passage of fluid through it. The superabsorbent material may be sodium polyacrylate and in the form of a powder which, advantageously, increases the surface area to volume ratio of the superabsorbent material, therefore enhancing the absorbent property of the material.

The non-woven fibres may be cellulose fibres. The cellulose non-woven fibres may be arranged as a cellulosic non-woven matrix of fibres and may be a support onto which the absorbent material may be incorporated, for example, in the described embodiment, the absorbent material is dispersed upon the cellulosic non-woven matrix. The absorbent material may be dispersed evenly, or substantially evenly, throughout or upon the non-woven fibres, for example the cellulosic non-woven matrix.

The upper layer and the lower layer of the superabsorbent layer 60 may each be a cellulosic fabric layer. The upper fabric layer and the lower fabric layer may be joined around their entire periphery to enclose the first material.

The superabsorbent layer 60 may comprise a plurality of fenestrations (not shown). Each of the plurality of fenestrations may be openings in the form of slits in the superabsorbent layer 60. Each fenestration may have a length equal to 80% of the length of the superabsorbent layer 60. Each of the upper layer and the lower layer may comprise a plurality of fenestrations. The fenestrations may be arranged in a direction planar to a longitudinal axis and transverse to a longitudinal axis of the superabsorbent layer 60. Advantageously, this arrangement aids in managing a flow of exudate through the superabsorbent layer 60 of the wound dressing 10. The fenestrations encourage or enable wound exudate to travel laterally on the superabsorbent layer 60, as opposed to axially, and assist in negative pressure transmission through the superabsorbent layer 60.

The superabsorbent layer 60 may have a basis weight of 460 g/m², a thickness of 2.55 mm and a density of 0.255 g/cm³. Moreover, the superabsorbent layer 60 may have a tensile strength (dry) of 45 N/50mm, an absorbent capacity of 25 g/g 10min, and an absorbency under compression capacity of 0.85 g/cm².

Each of the wound contact layer 40, transmission layer 50 and superabsorbent layer 60 are substantially planar.

In use, the wound dressing 10 forms part of a negative pressure wound exudate management system comprising the wound dressing 10 and further comprising a source of negative pressure (not shown) and a coupling member (for example, airway 80) for providing negative pressure to the wound dressing from an internal lumen of a conduit or tube (not shown) and through an opening 12 in the wound dressing 10. The source of negative pressure comprises a pump for generating negative pressure and the tube connects the pump and wound dressing 10 via the airway 80. In the present embodiment, the source of negative pressure may comprise a one-way valve (not shown) in-line between the pump and the wound dressing 10 to maintain a negative pressure within the wound dressing 10 when the pump is disconnected from the tube.

In use, the folded grip sections 21a of the removable cover 21 are separated and peeled in opposite directions to remove the removable cover 21 from the lower surface 200a of the adhesive skin contact layer 200. The adhesive skin contact layer 200 is therefore exposed and is then adhered to the intact peri-wound skin around a wound. The wound is located within the window 90 of the adhesive skin contact layer 200. The wound dressing 10 is connected to a source of negative pressure before or after application of the dressing to the wound. Upon leaving the wound, wound exudate contacts the first surface of the wound contact layer 40 and wicks away from the wound site, toward the transmission layer 50. The high gelling ability of the wound contact layer 40 facilitates absorption and retention of the wound exudate. Lamination of the wound contact layer 40, transmission layer 50 and superabsorbent layer 60 means each of these layers form a consolidated, absorbent and retentive structure 11. The wound exudate wicks from the upper surface of the wound contact layer 40 to the transmission layer 50. Lateral movement of the exudate occurs in the transmission layer 50 to spread the wound exudate across the surface area of the dressing. The exudate then wicks to the superabsorbent layer 60 where it is absorbed and retained prior to being transmitted out of the dressing 10 via vapour transmission through the backing film 70.

The one or more embodiments are described above by way of example only. Many variations are possible without departing from the scope of protection afforded by the appended claims.

## Claims

1. A negative pressure wound dressing (1, 10) comprising a backing layer (7, 70), an adhesive skin contact layer (2, 20, 200) and an absorbent structure (11) arranged between the backing layer (7, 70) and the adhesive skin contact layer (2, 20, 200), wherein the adhesive skin contact layer (2, 20, 200) is configured to detachably adhere the dressing (1, 10) to a dermal surface, wherein the backing layer (7, 70) comprises a coupling member (8, 80) configured to connect the dressing (1, 10) to a negative pressure source, wherein the absorbent structure (11) comprises a wound contact layer (4, 40) arranged to contact the wound when the adhesive skin contact layer (2, 20, 200) is adhered to the skin adjacent the wound, the wound contact layer (4, 40) having a first surface (4a) and a second surface (4b), wherein the first surface (4a) of the wound contact layer (4, 40) contacts the wound in use, wherein the first surface (4a) comprises warp stitches, and **characterised in that** the wound contact layer (4, 40) comprises a plurality of gel-forming fibres, the second surface (4b) comprises warp and weft stitches, and the first surface (4a) does not comprise any weft stitches.

2. A negative pressure wound dressing (1, 10) according to claim 1, wherein the second surface (4b) comprises at least 4.0 weft stitches /cm.

3. A negative pressure wound dressing (1, 10) according to any of claims 1 or 2, wherein the second surface (4b) comprises about 7.5 weft stitches /cm.

4. A negative pressure wound dressing (1, 10) according to claim 1, wherein the second surface (4b) comprises fewer than 1.0 weft stitches /cm.

5. A negative pressure wound dressing (1, 10) according to any preceding claim, wherein the wound contact layer (4, 40) has a weight of between 40gsm and 250gsm.

6. A negative pressure wound dressing (1, 10) according to claim 5, wherein the wound contact layer (4, 40) has a weight of between 60gsm and 200gsm.

7. A negative pressure wound dressing (1, 10) according to any preceding claim, wherein the warp stitches comprised in the first surface (4a) and/or the second surface (4b) have a pitch of between 1.0mm and 30.0mm.

8. A negative pressure wound dressing (1, 10) according to claim 7, wherein the warp stitches comprised in the first surface (4a) and/or the second surface (4b) have a pitch of between 6.0mm and 7.0mm.

9. A negative pressure wound dressing (1, 10) according to any preceding claim, wherein the wound contact layer (4, 40) comprises sodium carboxymethylcellulose fibres.

10. A negative pressure wound dressing (1, 10) according to claim 9, wherein the sodium carboxymethylcellulose fibres have a degree of substitution of between 0.25 carboxymethyl groups per glucose unit and 0.35 carboxymethyl groups per glucose unit.

11. A negative pressure wound dressing (1, 10) according to claim 10, wherein the sodium carboxymethylcellulose fibres have a degree of substitution of 0.30 carboxymethyl groups per glucose unit.

12. A negative pressure wound dressing (1, 10) according to any preceding claim, wherein the wound contact layer (4, 40) is stitched using lyocell yarn.

13. A negative pressure wound dressing (1, 10) according to any preceding claim, wherein the wound contact layer (4, 40) comprises multiple layers bonded together by stitch bonding or lamination.

14. A negative pressure wound dressing (1, 10) according to claim 13, wherein the multiple layers comprise a first layer and a second layer, wherein a first surface of the first layer contacts the wound in use, further wherein the first surface of the first layer does not comprise any weft stitches.

15. A negative pressure wound dressing (1, 10) according to claim 14, wherein the second layer comprises a first surface and a second surface, the first surface of the second layer being arranged opposite a second surface of the first layer, and further wherein the second surface of the first layer, and the first and second surfaces of the second layer, comprise weft stitches.

## Patentansprüche

1. Unterdruck-Wundverband (1, 10), aufweisend eine Trägerschicht (7, 70), eine klebende Hautkontaktschicht (2, 20, 200) und eine zwischen der Trägerschicht (7, 70) und der klebenden Hautkontaktschicht (2, 20, 200) angeordnete absorbierende Struktur (11), wobei die klebende Hautkontaktschicht (2, 20, 200) eingerichtet ist, um den Verband (1, 10) lösbar an einer Hautoberfläche anzuhaften, wobei die Trägerschicht (7, 70) ein Verbindungselement (8, 80) aufweist, das eingerichtet ist, um den Verband (1, 10) mit einer Unterdruckquelle zu verbinden, wobei die absorbierende Struktur (11) eine Wundkontaktschicht (4, 40) aufweist, die derart angeordnet ist, dass sie mit der Wunde in Kontakt ist, wenn die klebende Hautkontaktschicht (2, 20, 200) an der Haut neben der Wunde haftet, wobei die Wundkontaktschicht (4, 40) eine erste Oberfläche (4a) und eine zweite Oberfläche (4b) aufweist, wobei die erste Oberfläche (4a) der Wundkontaktschicht (4, 40) bei Gebrauch mit der Wunde in Kontakt ist, wobei die erste Oberfläche (4a) Kettstiche aufweist, und **dadurch gekennzeichnet, dass** die Wundkontaktschicht (4, 40) eine Mehrzahl von Gel-bildenden Fasern aufweist, die zweite Oberfläche (4b) Kett- und Schussstiche aufweist und die erste Oberfläche (4a) keine Schussstiche aufweist.

2. Unterdruck-Wundverband (1, 10) nach Anspruch 1, wobei die zweite Oberfläche (4b) mindestens 4,0 Schussstiche/cm aufweist.

3. Unterdruck-Wundverband (1, 10) nach einem der Ansprüche 1 oder 2, wobei die zweite Oberfläche (4b) etwa 7,5 Schussstiche/cm aufweist.

4. Unterdruck-Wundverband (1, 10) nach Anspruch 1, wobei die zweite Oberfläche (4b) weniger als 1,0 Schussstiche/cm aufweist.

5. Unterdruck-Wundverband (1, 10) nach einem der vorstehenden Ansprüche, wobei die Wundkontaktschicht (4, 40) ein Gewicht zwischen 40 g/m² und 250 g/m² aufweist.

6. Unterdruck-Wundverband (1, 10) nach Anspruch 5, wobei die Wundkontaktschicht (4, 40) ein Gewicht zwischen 60 g/m² und 200 g/m² aufweist.

7. Unterdruck-Wundverband (1, 10) nach einem der vorstehenden Ansprüche, wobei die in der ersten Oberfläche (4a) und/oder der zweiten Oberfläche (4b) enthaltenen Kettstiche einen Abstand zwischen 1,0 mm und 30,0 mm aufweisen.

8. Unterdruck-Wundverband (1, 10) nach Anspruch 7, wobei die in der ersten Oberfläche (4a) und/oder der zweiten Oberfläche (4b) enthaltenen Kettstiche einen Abstand zwischen 6,0 mm und 7,0 mm aufweisen.

9. Unterdruck-Wundverband (1, 10) nach einem der vorstehenden Ansprüche, wobei die Wundkontaktschicht (4, 40) Natriumcarboxymethylcellulosefasern aufweist.

10. Unterdruck-Wundverband (1, 10) nach Anspruch 9, wobei die Natriumcarboxymethylcellulosefasern einen Substitutionsgrad zwischen 0,25 Carboxymethylgruppen je Glucoseeinheit und 0,35 Carboxymethylgruppen je Glucoseeinheit aufweisen.

11. Unterdruck-Wundverband (1, 10) nach Anspruch 10, wobei die Natriumcarboxymethylcellulosefasern einen Substitutionsgrad von 0,30 Carboxymethylgruppen je Glucoseeinheit aufweisen.

12. Unterdruck-Wundverband (1, 10) nach einem der vorstehenden Ansprüche, wobei die Wundkontaktschicht (4, 40) mit Lyocellgarn vernäht ist.

13. Unterdruck-Wundverband (1, 10) nach einem der vorstehenden Ansprüche, wobei die Wundkontaktschicht (4, 40) mehrere Schichten aufweist, die mittels Nähgewirken oder Laminierung miteinander verbunden sind.

14. Unterdruck-Wundverband (1, 10) nach Anspruch 13, wobei die mehreren Schichten eine erste Schicht und eine zweite Schicht aufweisen, wobei eine erste Oberfläche der ersten Schicht bei Verwendung mit der Wunde in Kontakt ist, wobei ferner die erste Oberfläche der ersten Schicht keine Schussstiche aufweist.

15. Unterdruck-Wundverband (1, 10) nach Anspruch 14, wobei die zweite Schicht eine erste Oberfläche und eine zweite Oberfläche aufweist, wobei die erste Oberfläche der zweiten Schicht gegenüber einer zweiten Oberfläche der ersten Schicht angeordnet ist, und wobei ferner die zweite Oberfläche der ersten Schicht und die erste und zweite Oberfläche der zweiten Schicht Schussstiche aufweisen.

## Revendications

1. Un pansement (1, 10) de plaie à dépression comprenant une couche (7, 70) de support, une couche (2, 20, 200) adhésive de contact avec la peau et une structure (11) absorbante disposée entre la couche (7, 70) de support et la couche (2, 20, 200) adhésive de contact avec la peau, dans lequel la couche (2, 20, 200) adhésive de contact avec la peau est configurée pour faire adhérer de manière détachable le pansement (1, 10) à une surface dermique, dans lequel la couche (7, 70) de support comprend un élément (8, 80) d'accouplement configuré pour relier le pansement (1, 10) à une source de dépression, dans lequel la structure (11) absorbante comprend une couche (4, 40) de contact avec une plaie agencée pour être en contact avec la plaie, lorsque l'on fait adhérer la couche (2, 20, 200) adhésive de contact avec la peau à la peau au voisinage de la plaie, la couche (4, 40) de contact avec la plaie ayant une première surface (4a) et une deuxième surface (4b), dans lequel la première surface (4a) de la couche (4, 40) de contact avec la plaie est en contact avec la plaie en utilisation, dans lequel la première surface (4a) comprend des points de trame, et **caractérisé en ce que** la couche (4, 40) de contact avec la plaie comprend une pluralité de fibres formant du gel, la deuxième surface (4b) comprend des points de trame et de chaîne, et la première surface (4a) ne comprend pas de points de chaîne.

2. Un pansement (1, 10) de plaie à dépression suivant la revendication 1, dans lequel la deuxième surface (4b) comprend au moins 4,0 points de chaîne/cm.

3. Un pansement (1, 10) de plaie à dépression suivant l'une quelconque des revendications 1 ou 2, dans lequel la deuxième surface (4b) comprend environ 7,5 points de chaîne/cm.

4. Un pansement (1, 10) de plaie à dépression suivant la revendication 1, dans lequel la deuxième surface (4b) comprend moins de 1,0 points de chaîne/cm.

5. Un pansement (1, 10) de plaie à dépression suivant l'une quelconque des revendications précédentes, dans lequel la couche (4, 40) de contact avec la plaie a un poids compris entre 40 grammes par mètre carré et 250 grammes par mètre carré.

6. Un pansement (1, 10) de plaie à dépression suivant la revendication 5, dans lequel la couche (4, 40) de contact avec la plaie a un poids compris entre 60 grammes par mètre carré et 200 grammes par mètre carré.

7. Un pansement (1, 10) de plaie à dépression suivant l'une quelconque des revendications précédentes, dans lequel les points de trame compris dans la première surface (4a) et/ou la deuxième surface (4b) ont un pas entre 1,0 mm et 30,0 mm.

8. Un pansement (1, 10) de plaie à dépression suivant la revendication 7, dans lequel les points de trame compris dans la première surface (4a) et/ou la deuxième surface (4b) ont un pas compris entre 6,0 mm et 7,0 mm.

9. Un pansement (1, 10) de plaie à dépression suivant l'une quelconque des revendications précédentes, dans lequel la couche (4, 40) de contact avec la plaie comprend des fibres de carboxyméthylcellulose de sodium.

10. Un pansement (1, 10) de plaie à dépression suivant la revendication 9, dans lequel les fibres de carboxyméthylcellulose de sodium ont un degré de substitution compris entre 0,25 groupe carboxyméthyle par motif glucose et 0,35 groupe carboxyméthyle par motif glucose.

11. Un pansement (1, 10) de plaie à dépression suivant la revendication 10, dans lequel les fibres de carboxyméthylcellulose de sodium ont un degré de substitution de 0,30 groupe carboxyméthyle par motif cellulose.

12. Un pansement (1, 10) de plaie à dépression suivant l'une quelconque des revendications précédentes, dans lequel la couche (4, 40) de contact avec la plaie est cousue en utilisant du fil de lyocell.

13. Un pansement (1, 10) de plaie à dépression suivant l'une quelconque des revendications précédentes, dans lequel la couche (4, 40) de contact avec la plaie comprend de multiples couches reliées ensemble par couture ou stratification.

14. Un pansement (1, 10) de plaie à dépression suivant la revendication 13, dans lequel la couche multiple comprend une première couche et une deuxième couche, dans lequel une première surface de la première couche est en contact avec la plaie en utilisation, dans lequel en outre la première surface de la première couche ne comprend pas de points de chaîne.

15. Un pansement (1, 10) de plaie à dépression suivant la revendication 14, dans lequel la deuxième couche comprend une première surface et une deuxième surface, la première surface de la deuxième couche étant disposée à l'opposé d'une deuxième surface de la première couche, et dans lequel en outre la deuxième surface de la première couche et la première et la deuxième surfaces de la deuxième couche comprennent des points de chaîne.
